# EUROPEAN PATENT SPECIFICATION

(11) **EP 1 049 481 B1**
(45) Date of publication and mention of the grant of the patent: **20.04.2005**
(21) Application number: 98948504.0
(22) Date of filing: 23.09.1998
(51) Int. Cl.: C07K 11/00, C07K 7/02, C07K 1/00, C07K 1/02

(54) **SYNTHETIC ANTINEOPLASTIC AGENTS DERIVED FROM DOLASTATIN 15 AND METHODS OF MAKING SAME**
SYNTHETISCHE ANTINEOPLASTISCHE WIRKSTOFFE ABGELEITET VON DOLASTATIN 15 SOWIE VERFAHREN ZU IHRER HERSTELLUNG
AGENTS ANTINEOPLASIQUES SYNTHETIQUES DERIVES DE LA DOLASTATINE 15 ET LEURS PROCEDES DE FABRICATION

(30) Priority: 24.09.1997 US 59853 P
(43) Date of publication of application: 08.11.2000
(73) Proprietor: ARIZONA BOARD OF REGENTS, Tempe Arizona 85287 (US)
(72) Inventor: PETTIT, George, R., Paradise Valley, AZ 85253 (US); FLAHIVE, Erik, J., Tempe, AZ 85281 (US)
(74) Representative: Thomas, Philip John Duval
(86) International application number: PCT/US1998/019954
(87) International publication number: WO 1999/015130

(56) References cited:
- WO-A-93/23424
- WO-A-97/17364
- US-A- 5 654 399
- US-A- 5 663 149
- US-A- 5 780 588
- Bioorg. Med. Lett. 4(14):1947-1950(1994);Biochem. Pharmacol. 43(12):2637-2645(1992);Anti-Cancer Drug Design 10:529-544(1995)

## Description

Dolastatin 15, a known and potent antineoplastic constituent of the Indian Ocean shell-less mollusk *Dolabella auricularia*, was utilized as the lead substance from which were developed a series of novel derivatives. The present invention relates to methods of synthetically producing these new agents and presents their *in vitro* evaluations against a variety of murine and human cancer cell lines, and against a selection of bacteria and fungi. The effect of these derivatives on the inhibition of tubulin polymerization is also disclosed. Surprisingly, all of the new compounds, in which the C-terminal (S)-dolapyrrolidinone unit (Dpy, **5**) of Dolastatin 15 is replaced with a series of structurally diverse, more readily available and less expensive amides, show cancer cell growth inhibition activities which are quite comparable to those of Dolastatin 15 (see: U.S. Patent No. 4,879,278, Pettit et al All of the new compounds were, however, less potent than Dolastatin 15 as inhibitors of cubulin polymerization. The structurally modified peptides also caused mitotic arrest in cultured cells and inhibited the growth of a Gram-negative bacterium.

Some of this work was funded by Outstanding Investigator Grant CA-44344-01-08 awarded by the Division of Cancer Treatment, National Cancer Institute, DHHS. The United States government may have certain rights to this invention.

Marine organisms are an exceptionally productive source of biologically active and medicinally important substances bearing unique structures (see: FAULKNER, D. J. 1994, Marine Natural Products, *Natural Products Reports*, 11, 355; KOBAYASHI, M., *et al*. 1994, Bioactive substances isolated from marine sponge, a miniature conglomerate of various organisms, *Pure and Applied Chemistry*, 819; and, KÖNIG, G. *et al*. 1994, Biological activities of selected marine natural products, *Planta Medica*, 60, 532-537). Illustrative are the Indian Ocean (see: PETTIT et al. 1993, The isolation of dolastatins 10-15 from the marine mollusk *Dolabella auricularia, Tetrahedron*, 49, 9151) and Japanese (see: NAKAMURA *et al*., 1995, Stereochemistry and total synthesis of Dolastatin E. *Tetrahedron Letters*, 36, 5059, and the references cited therein) varieties of the sea hare *Dolabella auricularia*, from which a large number of antineoplastic and/or cytostatic linear and cyclic peptides, designated the dolastatins, have been isolated. Most of these potentially important peptides contain unprecedented amino acid units. Among these, the linear peptides, dolastatin 15 (**1**) (see: PETTIT et al., 1989a, Isolation and structure of the cytostatic linear depsipeptide dolastatin 15, *Journal of Organic Chemistry*, 54, 6005) and dolastatin 10 (**2**) (see: PETTIT et al., 1987, The isolation and structure of a remarkable marine animal antineoplastic constituent: Dolastatin 10, *Journal of the American Chemical Society,* 109, 6883) have exhibited the most potent antineoplastic activity (see: U.S. Patents 4,816,444; 4,879,278; 4,978,744 and 5,554,725; and Hu et al., 1993, Effects of dolastatins on human B-lymphocycic leukemia cell lines, *Leukemia Research*, 17, 333) and have been selected for clinical development. Indeed, Phase 1 clinical trials of dolastatin 10 (**2**) have been ongoing under the auspices of the U.S. National Cancer Institute since November, 1995.

Since 1984, considerable research efforts have been directed to exploring structural modifications of dolastatin 10 (**2**) (PETTIT *et al*., 1995, Antineoplastic Agents 337, Synthesis of Dolastacin 10 Structural Modifications, *Anticancer Drug Design*, 10, 529) and dolastatin 15 (**1**) for the purpose of developing new potential anticancer drugs. A number of structural modifications of these peptides have been investigated in order to alter the antineoplastic activity of the parent molecule and eliminate from each peptide where possible, the more synthetically challenging units, especially the phenylalanine-derived C-terminal segments. Preliminary structure/activity studies based on dolastatin 10 (**2**) suggested that the thiazole-containing C-terminal unit could be adequately replaced with β-phenethylamine without significant loss of activity, whereas certan other modifications led to moderate or more drastic loss of antiproliferative activity (see: PETTIT *et al*., 1995, Antineoplastic Agents 337, Synthesis of Dolastatin 10 Structural Modifications, *Anticancer Drug Design,* 10, 529) without significant change in inhibitory effects on tubulin assembly. In the case at hand, a series of structural modifications of dolastatin 15 were made in which the C-terminal dolapyrrolidinone unit (**5**) was replaced by various amides. In contrast to dolastatin 10, major structural changes in the C-terminal amide unit of dolastatin 15 had essentially no adverse affect upon the inhibitory effects of the depsipeptide against tumor cell growth, but did result in a moderate reduction in the inhibition of tubulin polymerization.

WO 93/23424 relates to novel derivatives of dolastatin 10 that have an antineoplastic effect, and describes the preparation thereof.

WO 97/17364 relates to novel peptides of dolastatin 15 that are suitable for controlling disease, and describe the preparation thereof.

One major factor driving the present research arises from the inescapable fact that there is not enough *Dolabella auricularia* in the world to allow sufficient quantities of effective components to be isolated therefrom to meet the need of the cancer-afflicted population in a commercially feasible manner. Therefore, a commercially effective synthesis must be developed which is capable of replicating a molecule containing only those substituents which are effective to control or arrest or mitigate the spread of cancer cells through a human system inflicted therewith. It is toward that goal that the present invention is directed.

### Materials and Methods

All amino acids (S-configurations) and derivatives which are discussed herein were used as obtained from Sigma-Aldrich Co. Other reagents (DEPC, DCC, EDC-HCl, HOBt, NMM, Et₃N, 4-pyrrolidinopyridine, TFA, etc. {Abbreviations used: DEPC (diethylphosphorocyanidate), DCC (*N, N'*-dicyclo-hexylcarbodiimide), EDC-HCl (1-ethyl-3-(3-dimethylaminopropyl)carbodiimide hydrochloride), WRK (Woodward's reagent K, 2-ethylphenylisoxazolium-3'-sulfonate), BroP (tris(dimethylamino)phosphonium bromide hexafluorophosphate), HOBt (1-hydroxybenzotriazole), NMM (4-methylmorpholine), Et₃N (triethylamine), TFA (trifluoroacetic acid), THF (tetrahydrofuran), EtOAc (ethyl acetate), AcOH (acetic acid), Z (benzyloxycarbonyl), Boc (*tert*-butyloxycarbonyl)}) described were also obtained from Sigma-Aldrich and used without further purification. Amines **6a-r** were either redistilled or recrystallized. All solvents were redistilled, and solvent extracts of aqueous solutions were dried over anhydrous magnesium sulfate or sodium sulfate. THF was distilled from LiAlH₄. Reactions were monitored by thin-layer chromatography using ANAL TECH silica gel GF (0.25 mm) plates visualized by either UV irradiation or 3% ceric sulfate in 3 N H₂SO₄ solution as appropriate. Crude products were purified by flash chromatography over silica gel (E. Merck, DARMSTADT, 70-230 mesh). The final peptide products (**12a-r**) were further purified by rapid gel permeation chromatography in methanol on a column of lipophilic SEPHADEX LH-20.

Melting points were measured with an ELECTROTHERMAL digital melting point apparatus, model IA9200, and are uncorrected. Optical rotation measurements were recorded on a PERKIN-ELMER 241 polarimeter in methanol (unless otherwise noted) at 25°C. IR spectra were obtained using a NICOLET FTIR Model MX-1 instrument. All ¹H-NMR spectra were observed on a VARIAN GEMINI 300 MHz instrument with CDCl₃ or DMSO-d₆ as solvent as noted. The ¹³C-NMR spectra were obtained with a UNITY 500 MHz instrument in CDCl₃. EIMS data were recorded with a MAT 312 mass spectrometer. Elemental analyses were determined by Galbraith Laboratories, Inc. located in Knoxville, TN.

All compounds synthesized were first evaluated for *in vitro* antitumor activity against murine P388 lymphocytic leukemia cells and against murine L1210 leukemia cells and human CA46 Burkitt lymphoma cells using techniques described by Hamel & Lin (see: HAMEL, E., and LIN, C.M., 1993, Interaction of combretastatin, a new plant-derived antimitotic agent, with tubulin, *Biochemical Pharmacology*, 32, 3864). The compounds were also evaluated in the NCI's human tumor 60-cell-line *in vitro* primary screen as described by Monks *et al*. (see: MONKS, A., SCUDIERO, *et al*., 1991, New colorimetric cytotoxicity assay for anticancer drug screening, *Journal of the National Cancer Institute*, 83, 757). Data analyses were performed using methods described by Boyd and Paull (see: BOYD, M.R., *et al*., 1995, Some practical considerations and applications of the National Cancer Institute *in vitro* anticancer drug discovery screen, *Drug Development Research*, 34, 91). A tubulin polymerization inhibition assay was performed according to a modified version of the procedure used previously and described by Muzaffar *et al*. (see: MUZAFFAR, A., *et al*., 1990, Antitubulin effects of derivatives of 3-demethylthiocolchicine, methylthio esters of natural colchicinoids, and thiokerones derived from thiocolchicine, Comparison with colchicine, *Journal of Medicinal Chemistry*, 33, 567) to evaluate the effects of dolastatin 15 on cellular microtubule assembly. The new assay for these compounds employed a drug-tubulin preincubation (15 min.) with 10 µM tubulin in 0.8 M glutamate at 30°C, followed by addition of GTP and incubation (20 min.) at 30°C, with tubulin polymerization monitored turbidimetrically in GILFORD recording spectrophotometers.

Antimicrobial disk susceptibility tests were performed according to the method established by the National Committee for Clinical Laboratory Standards (NCCLS, 1997). MUELLER-HINTON agar was used for susceptibility testing of *Staphylococcus aureus, Enterococcus faecalis, Micrococcus luteus, Escherichia coli, Enterobacter cloacae, Bacillus subtilis, Pseudomonas aeruginosa*, and *Erwinia carotovora*, Gonococcal Typing agar for *Neisseria gonorrhoeae*, and YM agar for *Candida albicans* and *Cryptococcus neoformans*. Immediately prior to susceptibility assays, compounds were reconstituted in sterile DMSO and twofold dilutions applied to sterile 6-mm disks. Zones of inhibition were read at 16 hours for bacterial cultures (except for *M. luteus*) and 42 hours for fungal cultures and *M. luteus*. MIC determinations were performed in duplicate.

### General Procedure for Synthesis of α-Hydroxy Amides 7a-r

### Method A (amides 7b-o)

N-*(2-Phenyl)ethyl-(2*S*)-2-hydroxyisovaleramide (7c)*. To a stirred and cooled (0°C) solution of (*S*)-(+)-Hiva (**3**, 2.0 g, 16.9 mmol), freshly redistilled 2-phenylethylamine (**6c**, 2.10 mL, 16.9 mmol), and NMM (1.86 mL, 16.9 mmol) in 30 mL dry CH₂Cl₂ is added DEPC (2.56 mL, 16.9 mmol) dropwise. The mixture is then allowed to reach room temperature over 2 hours and continuously stirred under Ar. The solution is washed with equal volumes of distilled H₂O (three times) and dried, and the solvent is removed *in vacuo* to give a yellow oil which crystallized. The product is recrystallized (three times) from toluene-hexane to give analytically pure amide **7c** as colorless needles (2.14 g, 58%). mp 100.2-100.5°C; R_{f} 0.28 (hexane-acetone, 2:1); [α]²⁴_{D} -42.0° (*c* = 1.0); MS *m*/*z* (relative intensity) 221 (48, M⁺), 168 (12), 148 (11), 130 (19), 104 (100), 91 (25), 73 (21), 55 (17); ¹H NMR (300 MHz, CDCl₃) δ 0.81 (d, *J* = 7.0 Hz, 3H, Val CH₃), 1.00 (d, *J* = 7.1 Hz, 3H, Val CH₃), 2.11 [spd (septet of doublets), *J*₁ = 7.0 Hz, *J*₂ = 3.3 Hz, 1H, Hiva CH_{β}], 2.49 (bs, 1H, -OH), 2.84 (t, *J* = 7.1 Hz, 2H, Ph-CH₂), 3.58 (m, *J* = 6.8 Hz, 2H, N-CH₂), 3.94 (d, *J* = 3.3 Hz, 1H, Hiva CH_{α}), 6.42 (bs, 1H, NH), 7.20-7.34 (m, 5H, ArH). Anal. Calcd for C₁₃H₁₉NO₂: C, 70.56; H, 8.65; N, 6.33. Found: C, 70.89; H, 9.01; N, 6.17.

### Method B (amides 7a, 7p-r)

**N***-(2-Benzothiazolyl)-(2***S***)-2-hydroxyisovaleramide (**7q**).* A solution of (S)-(+)-Hiva (**3**, 1.00 g, 8.5 mmol), 2-aminobenzothiazole (**6q**, 1.27 g, 8.5 mmol) and HOBt (2.29 g, 17 mmol, 2 eq) in dry THF (15 ml) is cooled to 0°C, under N₂, with stirring. To the solution is added NMM (0.93 mL, 8.5 mmol) and a solution of DCC (1.75 g, 8.5 mmol) in THF (5 mL). The reaction is then allowed to reach room temperature over 2 hours. The solution is filtered and concentrated to dryness. The oily residue is dissolved in CH₂Cl₂ (50 mL) and washed successively with saturated NaHCO₃ solution (50 mL), H₂O (50 mL), 10% citric acid solution (50 mL), and H₂O (2 X 50 mL). The organic extract is then dried and the solvent evaporated under reduced pressure to give a crude white solid. Urea byproduct is removed by repeated precipitations from ice-cold EtOAc, and the product is recrystallized (three times) from acetone-heptane to give the pure amide as colorless microcrystalline needles (1.8 g, 85%). mp 184.1-184.2°C; *R*_{*f*} 0.59 (CH₂Cl₂-EtOAc, 4:1); [α]²⁴_{D} -69.3° (*c* = 1.0); MS *m*/*z* 250 (9, M⁺), 151 (14), 150 (100), 123 (8), 96 (7), 82 (21), 80 (21), 73 (6), 55 (13); ¹H NMR (300 MHz, CDCl₃) δ 0.97 (d, *J* = 6.9 Hz, 3H, Val CH₃), 1.23 (d, *J* = 6.9 Hz, 3H, Val CH₃), 2.45 (spd, *J*₁ = 6.9 Hz, *J*₂ = 3.0 Hz, 1H, Hiva CH_{β}), 4.41 (bt, *J* = 3.0 Hz, 1H, Hiva CH_{α}), 5.00 (bd, *J* = 3.9 Hz, 1H, OH), 7.35 (t, *J* = 8.4 Hz, 1H, ArH), 7.47 (t, *J* = 8.4 Hz, 1H, ArH), 7.76 (d, *J* = 8.4 Hz, 1H, ArH), 7.85 (d, *J* = 7.5 Hz, 1H, ArH), 10.55 (bs, 1H, NH). Anal. Calcd. For C₁₂H₁₄N₂O₂S: C, 57.78; H, 5.64; N, 11.9. Found: C, 57.90; H, 5.87; N, 10.89.
For the physical properties of amides **7a-r**, see Table I, below.

**Table I**

| Physical constants for α-hydroxy amides **7a-r.** | | | | | |
|---|---|---|---|---|---|
| Compound | % yield^{a} | R_{f}^{b} | mp (°C) | ^{c}[α]_{D}²⁵ | MS |
| **7a** | 98 | 0.46 | 85.5 | -82.3° | 193 (M⁺), 151, 121, 94, 93, 77, 73, 66, 55 |
| **7b** | 57 | 0.46^{c} | 90.0-90.1 | -37.5° | 207 (M⁺), 189, 175, 165, 135, 106, 91, 73, 65, 55 |
| **7c** | 58 | 0.28 | 100.2-100.5 | -42.0° | 221 (M⁺), 168, 148, 130, 104, 91, 73, 55 |
| **7d** | 42 | 0.31 | 100.1-100.3 | -37.1° | 239 (M⁺), 196, 166, 130, 123, 122, 109, 83, 73, 55 |
| **7e** | 54 | 0.32 | 113.9-114.0 | -37.0° | 255 (M⁺), 212, 196, 182, 140, 138, 130, 89, 73, 55 |
| **7f** | 88 | 0.33^{c} | 87.9-88.0 | -33.1° | 255 (M⁺), 212, 182, 140, 139, 138, 130, 126, 103, 88, 77, 73, 55 |
| **7g** | 55 | 0.39^{c} | 54.9-55.0 | -47.3° | 255 (M⁺), 220, 214, 196, 182, 138, 137, 130, 89, 73, 55 |
| **7h** | 72 | 0.32 | 125.0-125.3 | -37.0° | 301 (M⁺), 299, 258, 184, 182, 169, 135, 130, 104, 90, 73, 55 |
| **7i** | 45 | 0.25 | 111.8-112.0 | -37.9° | 266 (M⁺), 223, 194, 177, 150, 137, 133, 121, 103, 91, 73, 55 |
| **7j** | 81 | 0.19 | 104.5-104.7 | -27.5° | 281 (M⁺), 164, 151, 137, 121, 107, 91, 73, 55 |
| **7k** | 53 | 0.49^{d} | 102.5 | -37.2° | 222 (M⁺), 179, 149, 121, 106, 93, 78, 66, 51 |
| **7l** | 80 | 0.31 | 61.0-61.4 | -35.6° | 235 (M⁺), 192, 162, 313, 118, 117, 113, 91, 83, 73, 55 |
| **7m** | 59 | 0.41^{c} | 73.2 | -23.9° | 279 (M⁺), 236, 220, 176, 162, 146, 131, 120, 91, 88, 73, 55 |
| **7n** | 70 | 0.35^{c} | 40.0-40.5 | -38.3° | 263 (M⁺), 202, 189, 171, 157, 139, 104, 89, 73, 61, 56, 55 |
| **7o** | 21^{f} | 0.37 | | | |
| **7p** | 57 | 0.28 | oil | -54.5° | 200 (M⁺), 127, 102, 101, 100, 76, 73, 58, 55 |
| **7q** | 85 | 0.59^{c} | 184.1-184.2 | -69.3° | 250 (M⁺), 151, 150, 123, 96, 82, 80, 73, 55 |
| **7r** | 46 | 0.39^{c} | 198.0-198.2 | -99.0° | 244 (M⁺), 173, 144, 128, 117, 89, 73, 55 |

| | | | | | |
|---|---|---|---|---|---|
| ^{a} Yields are for the pure isolated product after chromatography and/or recrystallization. | | | | | |
| ^{b} 2:1 hexane: acetone | | | | | |
| ^{c} 4:1 CH₂Cl₂-EtOAc | | | | | |
| ^{d} 9:1 CH₂Cl₂-CH₃OH ^{e} c = 1.0, CH₃OH | | | | | |
| ^{f} The yield of **7o** was low due to spontaneous diketomorpholine formation under the reaction conditions; the product was carried through to the subsequent step without characterization. | | | | | |

### General Procedure for Synthesis of Boc-Depsipeptides 8a-r

N*-[(2*S*)-O-[*N*-(tert-Butyloxycarbonyl)prolyl]-2-hydroxyisovaleryl]-2-phenylethylamide (8c)*. A solution of amide **7c** (0.50 g, 2.26 mmol) in CH₂Cl₂ (2 mL) is added slowly to a dry, stirred solution of *t*-Boc-*S*-proline (0.58 g, 2.71 mmol, 1.2 eq), 4-pyrrolidinopyridine (0.335 g, 2.71 mmol, 1.2 eq), and DCC (0.56 g, 2.71 mmol, 1.2 eq) in CH₂Cl₂. The solution is stirred for 20 hours at room temperature under Ar, filtered, and concentrated under reduced pressure to give an oil. The oily residue is then chromatographed on silica gel (25% acetone-hexane) to afford 0.95 g (100%) of ester **8c** as a colorless gum (after drying *in vacuo*). *R*_{f} 0.52 (hexane-acetone, 2:1); [α]²⁵_{D} -63.5° (*c* = 0.2); MS *m*/*z* 418 (16, M⁺), 362 (8), 327 (8), 271 (15), 170 (6), 142 (29), 114 (66), 105 (30), 91 (6), 70 (100), 57 (54); ¹H NMR (300 MHz, CDCl₃) δ 0.87 (d, *J* = 6.9 Hz, 3H, Val CH₃), 0.95 (d, *J* = 6.9 Hz, 3H, Val CH₃), 1.48 (s, 9H, *t*-butyl), 1.82-2.00 (m, 2H, Pro CH₂), 2.05-2.14 (m, 1H, ½ Pro CH₂), 2.22-2.31 (m, 1H, ½ Pro CH₂), 2.44 (spd, *J*₁ = 6.9 Hz, *J*₂ = 2.7 Hz, 1H, Hiva CH_{β}), 2.87 (t, *J* = 7.2 Hz, 2H, Ph-CH₂), 3.34-3.61 (m, 4H, Pro CH₂, N-CH₂), 4.39 (dd, *J*₁ = 8.7 Hz, *J*₂ = 4.2 Hz, 1H, Pro CH_{α}), 5.07 (d, *J* = 2.7 Hz, 1H, Hiva CH_{α}), 7.16-7.32 (m, 5H, ArH), 7.66 (bs, 1H, NH).
For the physical properties of compounds **8a-r**, see Table II.

**Table II**

| Physical constants for depsidipeptide derivatives **8a-r.** | | | | |
|---|---|---|---|---|
| Compound | % yield^{a} | R_{f}^{b} | ^{d}[α]_{D}^{Δ} | MS |
| **8a** | 81 | 0.49 | -65.5° | 390 (M⁺), 334, 289, 177, 162, 142, 114, 93, 70, 57 |
| **8b** | 92 | 0.51 | -41.5° | 404 (M⁺), 348, 189, 176, 170, 162, 114, 91, 70, 57 |
| **8c** | 100 | 0.52 | -63.5° | 418 (M⁺), 362, 327, 271, 170, 142, 114, 105, 91, 70, 57 |
| **8d** | 94 | 0.47 | -62.5° | 436 (M⁺), 380, 336, 271, 242, 170, 142, 115, 70, 57 |
| **8e** | 93 | 0.71^{c} | -82.5° | 452 (M⁺), 396, 352, 271, 242, 170, 142, 138, 114, 70, 57 |
| **8f** | 94 | 0.44 | -58.5° | 452 (M⁺), 396, 352, 271, 242, 170, 142, 114, 70, 57 |
| **8g** | 84 | 0.49 | -54.0° | 452 (M⁺), 396, 350, 327, 271, 242, 170, 142, 114, 70, 57 |
| **8h** | 84 | 0.46 | -53.5° | 496 (M⁺), 440, 396, 271, 242, 182, 142, 114, 70, 57 |
| **8i** | 93 | 0.36 | -56.0° | 463 (M⁺), 445, 352, 345, 271, 242, 170, 142, 114, 70, 57 |
| **8j** | 97 | 0.51 | -72.0° | 478 (M⁺), 422, 377, 264, 208, 164, 152, 114, 70, 57 |
| **8k** | 88 | 0.27 | -63.0° | 419 (M⁺), 346, 318, 277, 242, 204, 191, 149, 121, 114, 105, 93, 70, 57 |
| **8l** | 90 | 0.49 | -52.0° | 432 (M⁺), 376, 332, 290, 219, 204, 142, 114, 91, 70, 57 |
| **8m** | 100 | 0.49 | -118.5° | 476 (M⁺), 420, 375, 317, 242, 170, 162, 142, 114, 91, 70, 57 |
| **8n** | 88 | 0.48 | -92.0° | 460 (M⁺), 386, 359, 330, 300, 286, 162, 142, 116, 114, 70, 57 |
| **8o** | 58 | 0.43 | -132.5° | 426 (M⁺), 360, 325, 284, 242, 213, 198, 170, 142, 128, 114, 70, 57 |
| **8p** | 66 | 0.41 | -122.0° | 397 (M⁺), 296, 271, 215, 184, 160, 142, 127, 114, 100, 70, 57 |
| **8q** | 81 | 0.51 | -96.0° | 447 (M⁺), 346, 271, 235, 215, 177, 150, 142, 115, 114, 70, 57 |
| **8r** | 92 | 0.40 | -73.0° | 441 (M⁺), 340, 271, 228, 213, 171, 144, 142, 114, 70, 57 |

| | | | | |
|---|---|---|---|---|
| ^{a} Yields are tor the pure isolated product after chromatography. | | | | |
| ^{b} 2:1 hexane-acetone | | | | |
| ^{c} 4:1 CH₂Cl₂- EtOAc ^{d} c = 0.2, CH₃OH. | | | | |

### General Method for Deprotection of Boc-Depsipeptides 8a-r

To a cooled (0°C) and stirred solution of the appropriate dipeptide derivative (**8a-r**, 1 mmol) in CH₂Cl₂ (5 mL) is added TFA (5 mL) under an inert atmosphere. The solution is then stirred at this temperature for 1 hour, and the solvent is removed under reduced pressure. Residual TFA is removed azeotropically with toluene (3 X 20 mL), and the resulting oily trifluoroacetate salts (**9a-r**) are dried *in vacuo* and used directly in the subsequent reaction.

*Z- and Boc-valyl-*N*-methylvalyl-proline* (**10a,b**). The scaleup preparation of Z-Val-*N*-Me-Val-Pro (**10a**) was based on one of an earlier procedure reported by Dr. Pettit (see: PETTIT, G.R., *et al*. 1991, Antineoplastic agents 220, Synthesis of natural (-)-dolastatin 15, *Journal of the American Chemical Society*, 113, 6692). The same method used to synthesize Boc-Val-*N*-Me-Val-Pro (**10b**) is used here as is now described.

To a solution of *t*-Boc-valine (6.64 g, 30.5 mmol, 2 eq.) in dry CH₂Cl₂ (100 mL) cooled to -23°C under Ar is added NMM (6.70 mL, 61.1 mmol, 4 eq.) and pivaloyl chloride (3.76 mL, 30.5 mmol, 2 eq). After 3 hours at this temperature, a cooled (-23°C) solution of Me-Val-Pro-OMe (5.3 mmol; 3.7 g) in dry CH₂Cl₂ (15 mL) is slowly added. The reaction is allowed to proceed for 4 hours at this temperature and for 24 hours at room temperature. The reaction mixture is then washed with saturated citric acid solution (3 X 100 mL), water (100 mL), saturated NaHCO₃ solution (2 X 100 mL), and water (100 mL). The organic phase is dried and the solvent is evaporated to give a straw-colored oil. Flash chromatography (silica gel, 2.5" X 18" column, 15% acetone-hexane as eluent) led to 6.5 g (97%) of the pure dipeptide as a colorless oil: [α]_{D}²⁵ = -187° (*c* = 0.2). A 4.5-g (10.5 mmol) aliquot of the pure product is dissolved in 100 mL of 1:1 EtOH-H₂O, and 1 N NaOH (17 mL) is added. The mixture is vigorously stirred until saponification is complete as judged by TLC (2 hours), and the clear solution is concentrated to one-half volume under reduced pressure. The solution is then acidified to pH 3 with 1 N HCl, and the product is extracted with EtOAc (3 X 60 mL). The combined solvent extract is dried (Na₂SO₄) and concentrated *in vacuo* to give the pure tripeptide acid as a colorless glass (4.26 g, 98%). *R*_{f} 0.24 (hexane-EtOAc-AcOH, 8:2:1); [α]_{D}²⁵ = -188° (*c* = 0.2); MS *mlz* 427 (1, M⁺), 312 (3), 285 (3), 257 (2), 229 (4), 199 (7), 185 (5), 170 (4), 144 (5), 116 (23), 86 (100), 84 (21), 72 (28), 70 (14), 57 (35); ¹H NMR (300 MHz, CDCl₃) δ 0.86 (d, *J* = 6.6 Hz, 3H, Val CH₃), 0.91 (d, *J* = 6.6 Hz, 6H, 2 X Val CH₃), 0.97 (d, *J* = 6.9 Hz, 3H, Val CH₃), 1.42 (s, 9H, *t*-butyl), 1.84-2.40 (m, 6H, 2 X Val CH_{β}, 2 X Pro CH₂), 3.15 (s, 3H, N-CH₂), 3.62-3.71 (m, 1H, ½ Pro CH₂), 3.87-3.97 (m, 1H, ½ Pro CH₂), 4.42 (dd, *J*₁ = 9.3 Hz, *J*₂ = 7.2 Hz, 1H, Val CH_{α}), 4.49 (dd, *J*₁ = 7.5 Hz, *J*₂ = 6.3 Hz, 1H, Pro CH_{α}), 5.00 (d, *J* = 10.8 Hz, 1H, *N*-Me-Val CH_{α}), 5.22 (bd, *J* = 9.3 Hz, 1H, NH). Anal. Calcd. For C₂₂H₃₆N₃O₆: C, 59.84; H, 8.90; N, 9.52. Found: C, 59.61; H, 9.04; N, 9.34.

### General Procedure for Synthesis of Z- or Boc-depsipeptides 11a-r

N*-[(2*S*)-O-[[*N*-(Benzyloxycarbonyl)-valyl]-(*N*-methyl-valyl)-prolyl-prolyl]-2-hydroxyisovaleryl]-2-phenethylamide (***11*****c**)*. Amide **8c** (0.35g, 0.84 mmol) was deprotected in 1:1 TFA-CH₂Cl₂ (10 mL) at 0°C for 1 hour to yield trifluoroacetate salt **9c** as an oil (after drying *in vacuo* 1 hour). To a cooled (ice-bath) mixture of amide **9c** (0.27 g, 0.84 mmol) and Z-tripeptide **10a** (0.37 g, 0.80 mmol) in dry CH₂Cl₂ (10mL) is added Et₃N (0.23 mL, 1.68 mmol) and DEPC (0.13 mL, 0.88 mmol). The clear solution is stirred for 2 hours at 0°C, 18 hours at room temperature, and then concentrated. The product is separated by silica gel chromatography (1" X 16" column) with 1:3 acetone-hexane as eluent. The appropriate fractions are combined and concentrated to give the tetrapeptide (**11c**) as a colorless glass (0.61 g, 100%). *R*_{f} 0.43 (hexane-acetone, 1:1); [α]_{D}²⁵ -185° (*c* = 0.1); MS *m*/*z* 761 (0.5, M⁺), 6.53 (5), 562 (3), 499 (2), 442 (4), 416 (12), 347 (38), 324 (12), 239 (100), 211 (42), 196 (9), 139 (4), 109 (47); ¹H NMR (300 MHz, CDCl₃) δ 0.80-0.95 (m, 15H, 5 X Val CH₃), 1.02 (d, *J* = 6.6 Hz, 3H, Val CH₃), 1.82-2.35 (m, 10H, 2 X Val CH_{β}, 4 X Pro CH₂), 2.43 (spd, *J*₁ = 6.9 Mz, *J*₂ = 3.3 Hz, 1H, Hiva CH_{β}), 2.83 (t, *J* = 7.5 Hz, 2H, Ph-CH₂), 3.15 (s, 3H, N-CH₃), 3.35-3.52 (m, 2H, Pro CH₂), 3.55-3.70 (m, 2H, N-CH₂), 3.83-3.92 (m, 1H, ½ Pro CH₂), 3.96-4.06 (m, 1H, ½ Pro CH₂), 4.52 (dd, *J*₁ = 9.0 Hz, *J*₂ = 6.3 Hz, 1H, CH_{α}), 4.58-4.66 (m, 2H, 2 X CH_{α}), 5.07 (d, *J* = 12.0 Hz, 1H, N-Me-Val CH_{α}), 5.08 (d, *J* = 3.0 Hz, 1H, Hiva CH_{α}), 5.10 (s, 2H, Ph-CH₂-O), 5.42 (bd, *J* = 9.3 Hz, 1H, NH), 7.17-7.40 (m, 10H, ArH).
For the physical properties of compounds **11a-r**, see Table III, below.

**Table III**

| Physical constants for pentapeptide derivatives **11a-r**. | | | | |
|---|---|---|---|---|
| Compound | % yield^{a} | R_{f}^{b} | ^{d}[α]_{D}²⁵ | MS |
| **11a** | 86 | 0.42 | -199° | 733 (M⁺), 625, 583, 527, 471, 414, 291, 239, 211 |
| **11b** | 64 | 0.52 | -175° | 747 (M⁺), 639, 541, 485, 428, 402, 347, 305, 239, 211, 181, 139, 108 |
| **11c** | 100 | 0.43 | -185° | 761 (M⁺), 653, 562, 499, 442, 416, 347, 324, 239, 211, 196, 139, 109 |
| **11d** | 92 | 0.46 | -195° | 745 (M⁺), 645, 574, 517, 460, 434, 337, 313, 257, 239, 213, 181, 170, 142, 123, 115, 110 |
| **11e** | 91 | 0.39 | -208° | 795 (M⁺), 687, 589, 533, 476, 347, 324, 239, 211, 196, 181, 149 |
| **11f** | 84 | 0.46 | -161° | 761 (M⁺), 733, 690, 661, 590, 533, 477, 450, 407, 353, 313, 257, 239, 213, 198, 181, 170, 154 |
| **11g** | 95 | 0.43 | -192° | 761 (M⁺), 661, 636, 589, 533, 477, 450, 410, 353, 313, 257, 239, 213, 198, 181, 171, 154 |
| **11h** | 88 | 0.47 | -180° | 806 (M⁺), 707, 636, 579, 577, 522, 494, 397, 313, 257, 239, 213, 185, 181, 166, 154 |
| **11i** | 86 | 0.43 | -138° | 772 (M⁺), 636, 600, 584, 574, 410, 364, 313, 257, 239, 213, 198, 185, 181, 166, 154, 143, 117 |
| **11j** | 67 | 0.39 | -216° | 821 (M⁺), 713, 616, 615, 559, 502, 475, 379, 347, 239, 211, 164, 151, 108 |
| **11k** | 83 | 0.34 | -158° | 762 (M⁺), 654, 557, 500, 445, 417, 390, 374, 347, 277, 239, 211, 149, 121, 108 |
| **11l** | 83 | 0.49 | ^{e}-173° | 775 (M⁺), 667, 624, 569, 513, 456, 430, 347, 239, 211, 196, 181, 162 |
| **11m** | 91 | 0.28^{c} | -178° | 819 (M⁺), 711, 613, 557, 500, 474, 377, 347, 239, 162 |
| **11n** | 96 | 0.47 | -217° | 769 (M⁺), 695, 669, 597, 541, 527, 485, 458, 410, 383, 361, 313, 257, 239, 213, 198, 181, 142, 116 |
| **11o** | 78 | 0.33 | -289° | 769 (M⁺), 563, 507, 424, 347, 327, 239, 211, 162, 129 |
| **11p** | 98 | 0.52 | -193° | 706 (M⁺), 633, 606, 591, 535, 508, 478, 422, 410, 395, 367, 325, 313, 257, 239, 213, 198, 181, 170, 142, 116 |
| **11q** | 100 | 0.57 | -211° | 756 (M⁺), 683, 585, 528, 513, 472, 445, 417, 375, 313, 257, 239, 213, 198, 177, 150, 116 |
| **11r** | 99 | 0.23^{c} | -189° | 784 (M⁺), 676, 633, 579, 522, 466, 437, 396, 369, 347, 240, 227, 211, 213, 196, 171, 144, 125, 108 |

| | | | | |
|---|---|---|---|---|
| ^{a} Yields are tor the pure isolated product after chromatography. | | | | |
| ^{b} 1:1 hexane-acetone | | | | |
| ^{c} 2:1 hexane- acetone | | | | |
| ^{d} c = 0.1, CH₃OH | | | | |
| ^{e} c = 0.2, CH₃OH. | | | | |

### General Procedure for Deprotection of Z- or Boc-Depsipeptides 11a-r

### Method A (Z-Depsipeptides)

A solution of the appropriate Z-depsipeptide (0.1 mmol) in EtOAc (20 mL) is prepared, and 0.8 mmol of 10% Pd/C catalyst is added under a blanket of Ar. The solution is then vigorously stirred under hydrogen for 18 hours, and the catalyst is removed by filtration through CELITE. The resulting clear solution is concentrated *in vacuo* to give the corresponding free amine as a viscous oil, which is used directly in the subsequent coupling step.

### Method B (Boc-Depsipeptides)

To a cooled (0°C) and stirred solution of the Boc-terrapeptide derivative (0.5 mmol) in CH₂Cl₂ (2.5 mL) is added TFA (2.5 mL) under Ar. The solution is stirred at this temperature for 1 hour. Removal of solvent under reduced pressure affords an oil. Residual TFA is removed azeotropically with toluene (3 X 20 mL) to give the trifluoroacetate salt as a glassy solid which is dried *in vacuo* and used directly in the subsequent reaction step.

### Method C (Employing Z-Depsipeptrde 11e)

A 0.48-g sample of compound **11e** is suspended in 0.5 mL of glacial acetic acid, and 1.5 mL of 33% HBr/AcOH reagent is slowly added with stirring. After the evolution of CO₂ ceased (30 min), ethyl ether (15 mL) is added, and the solution is cooled. After several minutes, an oil, which is a mixture of deprotected peptide and unchanged starting material, separates from solution. The product is isolated and the ethereal solution is adjusted to pH ∼ 8 with saturated NaHCO₃ solution, followed by extraction with EtOAc (3 X 50 mL). The organic phase is concentrated to give a yellow glassy solid that is dried *in vacuo* and used without further purification in the next reaction.

### General Procedure for Coupling of dolavaline 4 With the Deprotected Depsipeptides to give new Dolastatin 15 Derivatives 12a-r

N*-[(2*S*)-*O*-[(*N,N-*Dimethyl-valyl)-valyl-(*N*-methyl-valyl)-prolyl-prolyl]-2-hydroxyisovaleryl]-2-phenethylamide (12c)*. Depsipeptide **11c** (0.37 g, 0.49 mmol) is deprotected by catalytic hydrogenolysis (Method A) to afford an oil, which is dissolved in CH₂Cl₂ (10 mL) and cooled to 0°C under Ar. To the solution is added dolavaline (**4**, 71 mg, 0.58 mmol), Et₃N (0.081 mL, 0.58 mmol) and DEPC (0.088 mL, 0.58 mmol), dropwise. After 2 hours, the solvent is removed under reduced pressure to yield an oil. Purification by flash chromatography (silica gel, 1" X 16" column, 3:2 hexane-acetone), is followed by rapid gel permeation chromatography on SEPHADEX LH-20 (CH₃OH elution) to provide a colorless foam (0.26 g, 71%). *R*_{f} 0.21 (hexane-acetone, 2:1); [α]_{D}²⁵ -206° (*c* = 0.1); MS *m*/*z* 754 (1, M⁺), 711 (2), 663 (0.5), 499 (0.5), 437 (3.5), 416 (5), 340 (32), 324 (5), 239 (4), 227 (7), 211 (5), 196 (9), 196 (10), 154 (5), 109 (9), 101 (59), 100 (100); IR (NaCl) νₘₐₓ 3312, 2963, 2934, 2874, 1744, 1638, 1535, 1497, 1443, 1279, 1204, 1186, 1096, 1038, 1003 cm⁻¹; ¹H NMR (300 MHz, CDCl₃) δ 0.78 (d, *J* = 6.6 Hz, 3H, Val CH₃), 0.83 (d, *J* = 6.6 Hz, 3H, Val CH₃), 0.89-0.98 (m, 12H, 5 X Val CH₃), 1.00 (d, *J* = 6.6 Hz, 6H, 2 X Val CH₃), 1.74-2.43 (m, 12H, Hiva CH_{β}, 3 X Val CH_{β}, 4 X Pro CH₂), 2.24 (s, 6H, 2 X Dov CH₃), 2.45 (d, *J* = 6.0 Hz, 1H, Dov CH_{α}), 2.82 (t, *J* = 7.8 Hz, 2H, Ph-CH₂), 3.20 (s, 3H, N-CH₃), 3.38-3.51 (m, 2H, Pro CH₂), 3.56-3.71 (m, 2H, N-CH₂), 3.83-3.94 (m, 1H, ½ Pro CH₂), 3.96-4.06 (m, 1H, ½ Pro CH₂), 4.59-4.68 (m, 2H, 2 X CH_{α}), 4.79 (dd, *J*₁ = 9.0 Hz, *J*₂ = 6.9 Hz, 1H, CH_{α}), 5.09 (d, *J* = 3.0 Hz, 1H, Hiva CH_{α}), 5.11 (d, *J* = 12.0 Hz, 1H, N-Me-Val CH_{α}), 6.89 (bd, *J* = 9.3 Hz, 1H, NH), 7.17-7.31 (m, 5H, ArH), 7.39 (bs, 1H, NH); ¹³C NMR (500 MHz, CDCl₃) δ 16.5, 17.6, 18.2, 18.6, 18.9, 19.2, 19.5, 20.2, 25.1, 25.2, 27.4, 27.7, 28.4, 29.3, 29.8, 30.7, 31.1, 35.4, 40.8, 43.0, 47.1, 47.8, 53.7, 57.9, 59.1, 59.6, 76.5, 78.6, 126.3, 128.4 (2), 128.7 (2), 139.0, 169.1, 169.4, 170.7, 171.5, 171.8, 173.2. Anal. Calcd for C₄₀H₆₆N₆O₇.½H₂O: C, 64.46; H, 8.84; N, 11.00. Found: C, 64.69; H, 9.00; N, 10.77.
For the physical properties of compounds **12a-r**, see Table IV.

**Table IV**

| Physical constants for dolastarin 15 structural modifications **12a-r.** | | | | |
|---|---|---|---|---|
| Compound | % yield^{a} | R_{f}^{b} | ^{d}[α]_{D}²⁵ | MS |
| **12a** | 87 | 0.32 | -234° | 726 (M⁺), 682, 526, 437, 415, 388, 340, 296, 255, 239, 227, 211, 197, 182, 170, 150 |
| **12b** | 92 | 0.49 | -183° | 740 (M⁺), 697, 485, 437, 429, 402, 347, 340, 239, 211, 197, 182, 154, 141, 139, 120, 106, 101, 100 |
| **12c** | 71 | 0.21^{c} | -206° | 754 (M⁺), 711, 663, 499, 437, 416, 340, 324, 239, 227, 211, 196, 154, 109, 101, 100 |
| **12d** | 79 | 0.27 | -215° | 772 (M⁺), 729, 663, 573, 437, 340, 324, 238, 227, 210, 196, 182 |
| **12e** | n.d. | 0.33 | -185° | 788 (M⁺), 745, 663, 589, 563, 533, 477, 450, 436, 340, 324, 255, 239, 227, 211, 196, 182 |
| **12f** | 83 | 0.27 | -179° | 788 (M⁺), 745, 663, 534, 475, 450, 437, 408, 340, 324, 296, 239, 227, 211, 206, 197, 182, 169, 154 |
| **12g** | 82 | 0.31 | -167° | 788 (M⁺), 745, 663, 585, 533, 476, 450, 408, 340, 324, 296, 239, 227, 211, 206, 197, 182, 169, 154 |
| **12h** | 84 | 0.29 | -177° | 834 (M⁺), 791, 663, 521, 494, 437, 340, 324, 312, 296, 239, 227, 211, 206, 197, 182, 169, 154 |
| **12i** | 90 | 0.28 | -183° | 799 (M⁺), 780, 756, 663, 600, 544, 530, 487, 461, 437, 354, 340, 324, 296, 239, 227, 211, 197, 182, 169, 154 |
| **12j** | 75 | 0.23 | -169° | 814 (M⁺), 771, 476, 437, 340, 239, 227, 211, 172, 164, 151, 101, 100 |
| **12k** | 65 | 0.19 | -163° | 755 (M⁺), 712, 437, 417, 339, 296, 277, 239, 227, 211, 196, 182, 149, 121, 108, 101, 100 |
| **12l** | 89 | 0.44 | ^{e}-164° | 768 (M⁺), 725, 569, 513, 433, 430, 340, 296, 262, 239, 226, 211, 196, 181, 155 |
| **12m** | 40 | 0.34 | -211° | 812 (M⁺), 770, 613, 557, 500, 474, 437, 414, 382, 340, 296, 239, 227, 211, 197, 182, 154, 141, 120, 101, 100 |
| **12n** | 82 | 0.35 | -132° | 796 (M⁺), 753, 722, 484, 458, 437, 340, 239, 227, 211, 198, 182, 154, 101, 100 |
| **12o** | 78 | 0.21 | -292° | 762 (M⁺), 719, 507, 451, 436, 424, 364, 339, 296, 255, 241, 227, 211, 197, 182, 164, 154 |
| **12p** | 72 | 0.34 | -174° | 733 (M⁺), 690, 437, 395, 340, 296, 239, 227, 211, 198, 182, 154, 127, 101, 100 |
| **12q** | 80 | 0.35 | -181° | 783 (M⁺), 740, 548, 528, 514, 472, 457, 445, 437, 417, 375, 340, 239, 227, 211, 198, 182, 177, 150, 101, 100 |
| **12r** | 91 | 0.29 | -192° | 777 (M⁺), 734, 466, 437, 369, 340, 296, 239, 227, 211, 197, 182, 171, 144, 128, 114, 101, 100 |

| | | | | |
|---|---|---|---|---|
| ^{a} Yields are tor the pure isolated product atter chromatography. | | | | |
| ^{b} 1:1 hexane-acetone | | | | |
| ^{c} 2:1 hexane- acetone | | | | |
| ^{d} c = 0.1, CH₃OH | | | | |
| ^{e} c = 0.2, CH₃OH. | | | | |

Both dolastatin 10 (see: BAI, R., *et al*., 1990a, Dolastatin 10, a powerful cytostatic peptide derived from a marine animal: Inhibition of tubulin polymerization mediated through the vinca alkaloid binding domain, *Biochemical Pharmacology*, 39, 1941) and dolastatin 15 (see: BAI *et al*., 1992, Dolastatin 15, a potent antimitotic depsipeptide derived from *Dolabella auricularia*: Interaction with tubulin and effects on cellular microtubules, *Biochemical Pharmacology*, 43, 2637) interact with tubulin and cause arrest of cellular mitosis. Effects on tubulin *in vitro* with dolastatin 10 have been studied in detail (see: BAI *et al*., 1990b; Binding of dolastarin 10 to tubulin at a distinct site for peptide antimitotic agents near the exchangeable nucleotide and vinca alkaloid sites, *Journal of Biological Chemistry*, 265, 17141; and BAI *et al*., 1995, Interaction of dolastatin 10 with tubulin: Induction of aggregation and binding and dissociation reactions, *Molecular Pharmacology*, 47*,* 965). Since the interaction of dolastatin 15 with tubulin appears considerably weaker, less attention has been given to the biochemical properties of this depsipeptide. Nevertheless, a number of studies have described the antiproliferative effects of dolastatin 15 against human lymphoma cells (see: BECKWITH *et al*., 1993, Growth inhibition of human lymphoma cell lines by the marine natural products dolastatins 10 and 15, *Journal of the National Cancer Institute*, 85, 483) on murine bone marrow progenitor cells (see: JACOBSEN *et al*., 1991, Antineoplastic dolastatins: Potent inhibitors of hematopoietic progenitor cells, *Journal of the National Cancer Institute*, 83, 1672), and on malignant peripheral blood cells from patients with acute myeloid leukemia (see: STEUBE *et al*., 1992, Dolastatin 10 and dolastatin 15: Effects of two natural peptides on growth and differentiation of leukemia cells, *Leukemia*, 6, 1048).

All of these investigations were made possible by the synthesis (see: PETTIT *et al*., 1991, Antineoplastic agents 220, Synthesis of natural (-)-dolastatin 15, *Journal of the American Chemical Society*, 113, 6692) of dolastatin 15 at the Cancer Research Institute in Tempe, Arizona which provided the first synthetic specimens of the peptide available anywhere. A subsequent, modified synthetic procedure (see: PETTIT *et al*., 1994, The dolastatins, 20, A convenient synthetic route to dolastatin 15, *Tetrahedron*, 50, 12097) proved very practical for large-scale preparation of this promising anticancer drug. Still another synthesis of dolastatin 15 derived subsequent to Pettit's original synthesis, is described by Patino *et al*. (see: PATINO *et al*., 1992, Total synthesis of the proposed structure of dolasrarin 15, *Tetrahedron*, 48, 4115-4122). The potential advantage of identifying suitable substitutions for the Dpy unit of dolastatin 15 became apparent at the start of the efforts to synthesize a commercially viable alternative. Most significant was the potential exclusion of six synthetic steps, and the elimination of the unpredictable dolapyrrolidinone cyclization sequence. Thus it was determined to replace the modified amino acid unit with readily available arylalkylamines based on β-phenethylamine, and with various other groups.

For the preparation of α-hydroxy amides **7a-r,** it was determined most efficient to obtain these substances without protection of the hydroxyl group. For the aliphatic amines **6b-o,** this objective was readily met by using Pettit's original synthetic approach (op. cit.). Thus 2-(S)-hydroxyisovaleric acid (Hiva, **3**) and the appropriate amine were condensed by use of DEPC with NMM as base to give compounds **7b-o** in good yield as shown in Scheme 1. Owing to the reduced nucleophilicity of the amino component, acylation of the aromatic amines (**6a, 6p-r**) with activated Hiva was more challenging. Mild coupling reagents such as DEPC (see: YAMADA *et al*., 1975, Diphenyl phosphorazidate (DPPA) and diethyl phosphorocyanidate (DEPC), Two new reagents for solid-phase peptide synthesis and their application to the synthesis of porcine motilin, *Journal of the American Chemical Society,* 97, 7174); WRK (see: PETTIT *et al*., 1966, Structural biochemistry II, Synthesis of 3β-Hydroxy-17β-(L-prolyl-L-prolyl)amino-5α-androstane, *Ganadian Journal of Chemistry*, 44, 2023; and PETTIT *et al*., 1967, Synthesis of 3β-Acetoxy-17β-(L-arginyl-L-arginyl-L-prolyl)amino-5α-androstane, *J*. *Med. Chem*., 10, 145) produced no reaction, whereas strong activation with DCC, BrOP (see: COSTE *et al*., 1990, A new reagent for coupling N-methylated amino acids, *Tetrahedron Letters*, 31, 669) or with various chloroformates (see: VAN BOGART *et al*., 1993, Synthesis and antitrypanosomal evaluation of some thiazole-containing amino acids and peptides, *European Journal of Medicinal Chemistry*, 28, 387; and NEDEV *et al*., 1993, A convenient method for synthesis of Fmoc-amino acid p-nitroanilides based on isobutyl chloroformate as condensation agent, *Tetrahedron Letters*, 34, 4201) allowed competitive esterification of the unprotected α-hydroxyl group, giving rise to complex mixtures. However, the HOBt ester of Hiva, prepared *in situ* with DCC at 0°C in anhydrous THF, was found to be very suitable for selective acylation of such aromatic amines. Indeed, the result was moderate to good yields of the desired amide with no trace of esterification side-products. In the subsequent step, Boc-proline was esterified with alcohols **7a-r** by use of DCC in CH₂Cl₂ with 4-pyrrolidinopyridine as catalyst (room temperature, 18 hours) to afford excellent yields of esters **8a-r** as viscous oils. It was then discovered that the water-soluble carbodiimide EDC-HCl worked equally well and gave fewer purification problems than DCC. The Boc protecting group was removed with TFA to yield the corresponding trifluoroacetates **9a-r.**

For the synthesis and subsequent deblocking of depsipeptides **11a-r,** it was first thought to use the Z- protection scheme which was first described in the original synthesis of dolastatin 15 (see U.S. Patent No. 4,879,278). However, the conditions of catalytic hydrogenolysis used to remove this group proved unsuitable for several of the peptide intermediates as reported below. Consequently, Boc-Val-*N*-Me-Val-Pro (**10b**) was synthesized in addition to the usual Z-tripeptide intermediate (**10a**), in order to circumvent catalytic hydrogenolysis. The DEPC-mediated segment condensation of the appropriate tripeptide (**10a** or **10b**) with trifluoroacetates **9a-r** gave consistently high yields of the corresponding depsipeptide derivative (**11a-r**) as shown in Scheme 2.

The mild catalytic hydrogenolysis conditions previously used to remove the Z-protecting group were found to cause quantitative dechlorination of compound **11e** and therefore forced the development of an alternative route for the halogenated aromatic amides **9d-h**. Although attempted deprotection of peptide **11e** with 33% hydrogen bromide in glacial acetic acid under the usual conditions caused no loss of halogen, extensive racemization of at least one of the amino acid units was observed, which quite likely is the strong-acid sensitive *N*-Me-Val unit, as reported by Benoiton *et al*. (see: BENOITON *et al*., 1973, N-methylamino acids in peptide synthesis, III, Racemization during deprotection by saponification and acidolysis, *Canadian Journal of Chemistry*, 51, 2555). Thus, all remaining peptides that contained halogenated aromatic rings were prepared as Boc-protected depsipeptides (**11d, 11f-h**). Deprotection of these peptides in TFA-CH₂Cl₂ (1:1) at 0°C for 1 hour proceeded smoothly and gave quantitative yields of the corresponding trifluoroacetate salts (used directly in the subsequent coupling reactions). The Boc- strategy was also utilized for the *p*-nitro-phenethylamine (**11i**), Met-OMe (**11n**), thiazole (**11p**), and benzothiazole (**11q**) amides in order to avoid unwanted nitro group reduction or catalyst poisoning by the sulfur-containing peptides. Deprotection of all other peptide intermediates by catalytic hydrogenolysis (10% Pd/C, EtQAc, 18 hours) gave the corresponding amines in excellent yields.

Final coupling of each peptide to the *N*-terminal dolavaline residue was accomplished in good yield with DEPC/Et₃N in CH₂Cl₂ as shown in Scheme 3. In some cases, owing to the basic nature of the final peptides **12a-r,** further purification is necessary following silica gel chromatography. In such cases, rapid gel permeation chromatography (SEPHADEX LH-20 methanol elution) gave very satisfactory results, affording the final peptides as colorless foams or amorphous powders.

Results of *in vitro* testing of the synthetic derivatives (**12a-r**) against the murine leukemia cell lines P388 and L1210 and the human Burkitt lymphoma CA46 line, along with the corresponding tubulin anti-polymerization results, are reported in Table V, below.

**Table V**

| Inhibitory activities of dolastatin 15 (**1**) and synthetic modifications (**12a-r**) on murine P388 lymphocytic leukemia [ED₅₀ (ng/mL)]^{a}, murine L1210 leukemia [IC₅₀ (nM)]^{b}, human CA46 Burkitt lymphoma [IC₅₀ (nM)]^{b,c}, and tubulin polymerization (IC₅₀ (:M) ± [SD]). | | | | | |
|---|---|---|---|---|---|
| Compound | Mouse Leukemia P388 | Mouse Leukemia L1210 | Human CA46 Burkitt Lymphoma | % Mitotic Cells | Tubulin Polymerization |
| **1** | 2.4 | 4 | 2 | 31 | 5.2 [0.6] |
| **12a** | 0.52 | 3 | 2 | 26 | 26 [6] |
| **12b** | 0.42 | 1 | 0.3 | 46 | 20 [3] |
| **12c** | 0.26 | 2 | 0.3 | 45 | 15 [1] |
| **12d** | 0.55 | 4 | 0.8 | 46 | 12 [2] |
| **12e** | 0.49 | ^{d} | ^{d} | ^{d} | ^{d} |
| **12f** | 0.37 | 4 | 2 | 38 | 14 [2] |
| **12g** | 0.33 | 4 | 2 | 26 | 10 [2] |
| **12h** | 0.36 | 2 | 2 | 48 | 11 [2] |
| **12i** | 0.60 | 4 | 3 | 36 | 11 [2] |
| **12j** | 0.40 | 2 | 0.8 | 31 | 21 [2] |
| **12k** | 0.40 | 4 | 2 | 30 | 20 [3] |
| **12l** | 0.38 | ^{d} | ^{d} | ^{d} | ^{d} |
| **12m** | 0.54 | 3 | 1 | 40 | 13 [3] |
| **12n** | 0.55 | 3 | 3 | 44 | 18 [4] |
| **12o** | 3.1 | 4 | 2 | 42 | 24 [2] |
| **12p** | 0.26 | 2 | 4 | 40 | 8.4 [1] |
| **12q** | 0.45 | 3 | 2 | 36 | 9.3 [2] |
| **12r** | 0.29 | 2 | 2 | 42 | 16 [2] |

| | | | | | |
|---|---|---|---|---|---|
| ^{a} ED₅₀ (P388) and IC₅₀ (L1210) refer to the drug dosages required to inhibit tumor cell growth by 50%. There is no mathematical difference between the two values which were both calculated identically in the present study. | | | | | |
| ^{b} L1210 cells were counted after 24 hours of growth, CA46 cells after 20 hours. | | | | | |
| ^{c} CA46 cells were treated with 80 nM drug for 20 hours; cells were harvested by centrifugation, fixed, and stained with Giemsa; control cells: 3% mitotic cells. | | | | | |
| ^{d} Compounds **12e** and **12I** were not evaluated in this assay. | | | | | |

All of the derivatives showed antiproliferative activities quite comparable to the performance of the parent dolastatin 15 (**1**) and, in the Burkitt cells, all analogues caused a marked rise in the mitotic index. None, however, was as potent an inhibitor of tubulin polymerization as dolastatin 15 (**1**). Likewise, repetitive testing of **12a-r** in the NCI's 60-cell-line human tumor primary screen consistently yielded mean panel GI₅₀ values of similar potency to that of the dolastatin 15 standard as shown in Table VI, below.

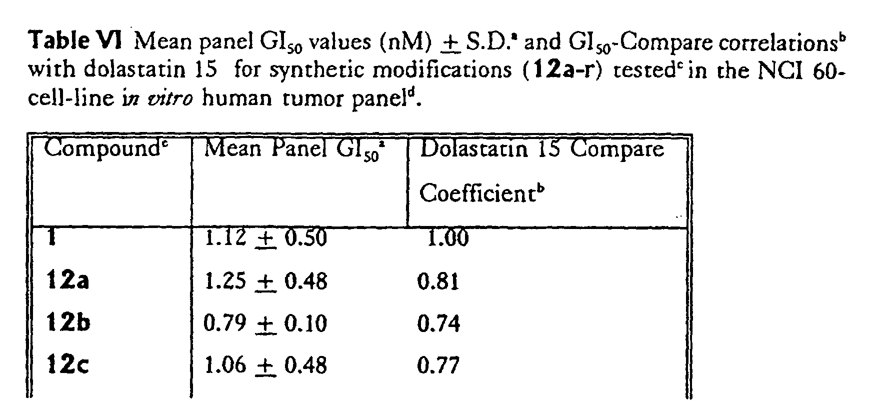

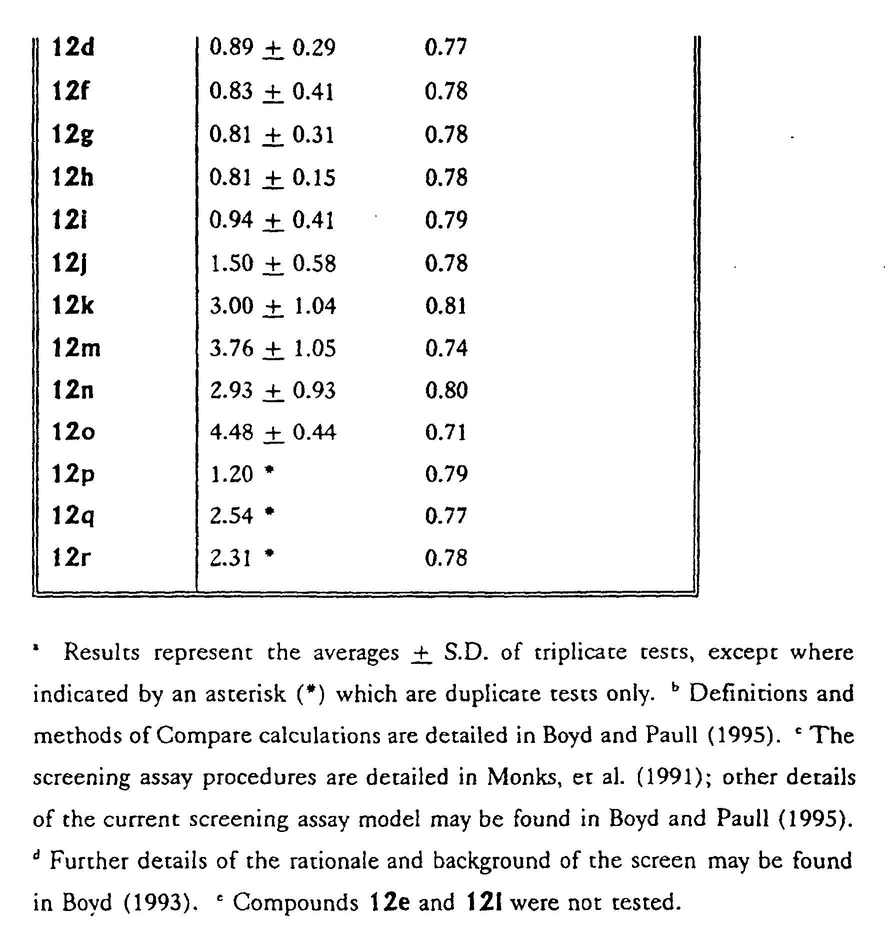

Moreover, using the COMPARE analyses described by Boyd and Paull (see: BOYD, *et al*., 1995, Some practical considerations and applications of the National Cancer Institute *in vitro* anticancer drug discovery screen, *Drug Development Research*, 34, 91), the mean graph fingerprints of **12a-r** in the NCI screen were all highly correlated (e.g., ≥ 0.7) with that of dolastatin 15. Thus, it was determined that the antiproliferative and antimitotic activities of dolastatin 15 are remarkably tolerant of changes in the C-terminal residue, whereas their antitubulin properties are more sensitive to such changes. These results are in contrast to analogous studies reported by Pettit *et al*. (see: PETTIT *et al*., 1995, Antineoplastic Agents 337, Synthesis of Dolastatin 10 Structural Modifications, *Anticancer Drug Design*, 10, 529) in which those derivatives of dolastatin 10 which had the C-terminal dolaphenine unit replaced with groups not containing a β-phenethylamine carbon skeleton displayed small differences in antitubulin potencies but encountered up to 100-fold decreases in cytotoxicity.

The interaction of dolastatin 15 with purified tubulin is considerably weaker than that of dolastatin 10 (about 20-fold) despite its potent antiproliferative effect on cellular microtubules (one-fourth as active as dolastatin 10, but ten times more active than the antitumor drug vinblastine) (see: BAI *et al*., 1992, Dolastatin 15, a potent antimitotic depsipeptide derived from *Dolabella auricularia:* Interaction with tubulin and effects on cellular microtubules, *Biochemical Pharmacology*, 43, 2637). In previous original studies on the antitubulin properties of dolastatin 15 reported by Bai *et al*. (see: *Id*.), the effects of this depsipeptide on the polymerization of 10 µM tubulin in 1 M monosodium glutamate-1 mM MgCl₂ were examined and provided an IC₅₀ value of 23 µM (50% inhibition of extent of polymerization after 20 min at 37°C). This result was confirmed in new experiments, where a value of 19± 0.3 (S.D.) µM was obtained. However, no IC₅₀ value was obtained for any of the dolastatin 15 derivatives presented herein under the reaction condition described, although clear inhibition of assembly rates did occur with these compounds (maximum concentration used, 40 µM).

With an increasing number of antimitotic drugs, particularly those that bind at the colchicine site, the tubulin polymers formed had a highly aberrant morphology in 1 M glutamate-1 mM MgCl₂ (see, for example, HAMEL *et al*., 1995, Limitations in the use of tubulin polymerization assays as a screen for the identification of new antimitotic agents: The potent marine natural product curacin A as an example, *Drug Development Research*, 3). Since these high molecular weight polymers, like microtubules, scatter light, they make it impossible to quantify drug effects by turbidimetry or centrifugation. In exploring alternate assay conditions for such compounds, it was discovered that the aberrant polymers were generally not formed in 0.8 M glutamate at 30°C (no MgCl₂ added to reactions). Under this reaction condition the normal assembly reaction still occurred at a reduced reaction rate, and there was a substantial reduction in the IC₅₀ value obtained with agents that could be examined under both reaction conditions.

An aberrant polymer formation was not observed with dolastatin 15, even though such a reaction does occur with dolastatin 10 (see: BAI *et al*., 1995, Interaction of dolastatin 10 with tubulin: Induction of aggregation and binding and dissociation reactions, *Molecular Pharmacology*, 47, 965). Nevertheless, when dolastatin 15 was evaluated for inhibition of assembly in 0.8 M glutamate at 30°C, the reduced IC₅₀ value effect was again observed. A value of 5.2± 0.6 µM was obtained. More importantly, with this reaction condition it was possible to perform a quantitative comparison of the dolastatin 15 analogue series described here (see Table V). Although none of the 16 compounds evaluated had an IC₅₀ value as low as that of dolastatin 15, unambiguous inhibitory effects were observed with all agents examined. The IC₅₀ values obtained ranged from 8.4 µM for compound **12p** to 26 µM for **12a**. Including dolastatin 15, there was a five-fold range in the IC₅₀ values obtained in this sequence of experiments.

The study of the interaction of dolastatin 15 with tubulin was performed in conjunction with an evaluation of the effects of the agent on the growth of murine L1210 leukemia cells and human CA46 Burkitt lymphoma cells (see: BAI *et al*., 1992, Dolastatin 15, a potent antimitotic depsipeptide derived from *Dolabella auricularia*: Interaction with tubulin and effects on cellular microtubules, *Biochemical Pharmacology*, 43, 2637). The effects of most of these new dolastatin 15 dervatives were examined on these same cell lines, and little difference was found between the compounds as reported in Table V.

With the L1210 cells, IC₅₀ values for cell growth were in the 1-4 nM range, and with the Burkitt cells the range of IC₅₀ values was 0.3-4 nM. Although the range of values observed was similar to the five-fold variation in IC₅₀ values for tubulin polymerization, there was little correlation between the values obtained for specific compounds. Thus, for example, dolastatin 15 was the most potent inhibitor of tubulin assembly, but it was among the least active compounds with the L1210 and Burkitt cells.

To further verify the assumption that this entire series of compounds is acting intracellularly by interfering with tubulin function, the drug-treated Burkitt cells for mitotic arrest were analyzed. As was observed previously for dolastatin 15 (see: BAI *et al*., 1992, Dolastatin 15, a potent antimitotic depsipeptide derived from *Dolabella auricularia*: Interaction with tubulin and effects on cellular microtubules, *Biochemical Pharmacology*, 43, 2637), cells treated with all compounds at 80 nM caused the accumulation of high numbers of Burkitt cells in apparent metaphase arrest (Table V).

It has been suggested that the potent cytotoxicity and tubulin inhibitory effects of dolastatin 15 are not due to the parent molecule, but to a more active metabolite derived therefrom. For instance, intracellular hydrolysis of the ester bond of the depsipeptide might produce a more potent peptide fragment or a fragment that is further metabolized to a more active species. This possibility seems consistent with the apparent insensitivity of dolastatin 15 activity to structural modification of the C-terminus and the recent observation by Roux, *et al*. (see: ROUX *et al*., 1994, Synthesis and *in vitro* cytotoxicity of diastereomerically modified dolastatin 15 analogues, *Bioorganic and Medicinal Chemistry Letters*, **4,** 1947) that changes in the stereochemistry of the Hiva-Dpy unit are attended by minimal loss of cytotoxicity. Especially interesting here is the observation that the peptide segment Dov-Val-Me-Val-Pro-Pro is apparently inactive while the synthetic dolastatin 15 derivative LU103793 reported by Dearruda *et al*. (see: DEARRUDA *et al*. 1995, LU103793 (NSC D-669356): A synthetic peptide that interacts with microtubules, *Cancer Research*, **55,** 3085), Dov-Val-Me-Val-Pro-Pro-benzamide (NSC 669356), is both highly cytotoxic (IC₅₀, 3 nM against L1210 leukemia cells) and a potent inhibitor of tubulin polymerization (IC₅₀, 4.0± 0.6 µM) under the same reaction conditions in which dolastatin 15 was 50% inhibitory at 5.2 µM. Alternatively, the benzamide derivative LU103793 may be necessary for proper transport of the peptide to the site(s) of activity.

It is now clear that the structurally unique Dpy unit of dolastatin 15 plays a very limited role in the cell growth inhibitory effects observed for dolastatin 15 and is probably not a critical component of the active site(s) of dolastatin 15. This fact presents an unusual opportunity to further enhance the ability to achieve the benefits of dolastatin 15 with a much simpler and cost effective manner and to optimize or otherwise exploit the *in vivo* antitumor effect of these unique compounds. It is thus apparent that the complex Dpy unit of dolascacin 15 can be readily replaced by amides derived from the more readily available phenethylamine, 2-aminothiazole, and 2-aminobenzothiazole, or by other relatively available and innocuous substituents without any significant diminution of cancer cell growth inhibitory activity of the resultant derivative.

Of nine bacteria and two fungi screened, dolastatin 15 and the eight derivatives described herein specifically inhibited growth of the enteric bacterium *Erwinia carotovora* (Table VII). The parent compound was the most active against *E. carotovora*.

**Table VII**

| Minimum inhibitory concentrations (µg/disk) of dolastatin 15 and structural modifications for the bacterium *Erwinia carotovora* | |
|---|---|
| Peptide | *Erwinia carotovora* |
| Dolastatin 15 | 3.12 - 6.25 |
| **12b** | 50-100 |
| **12c** | 50-100 |
| **12j** | 50-100 |
| **12k** | 50-100 |
| **12m** | 50-100 |
| **12p** | 50-100 |
| **12q** | 50-100 |
| **12r** | 50-100 |

As used here, the bold numerals **1, 2** etc. refer to those compounds whose structures are shown below.

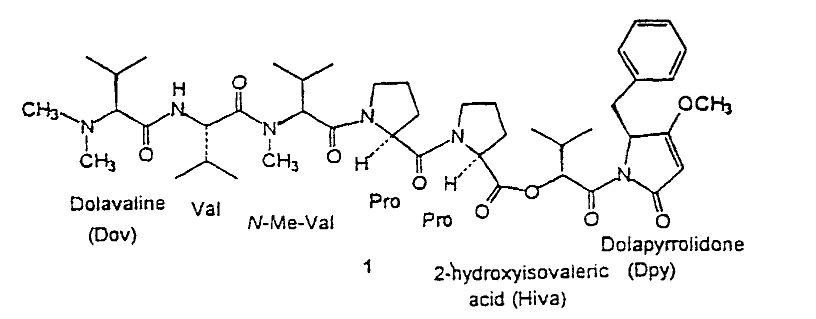

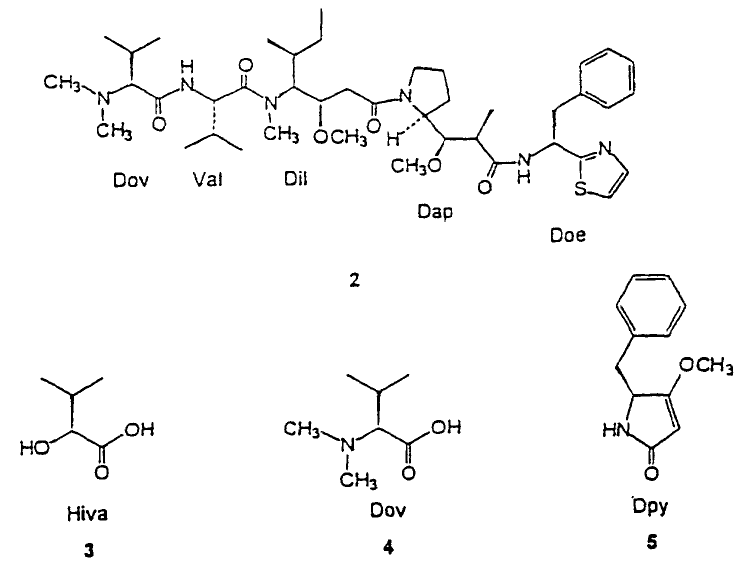

The synthesis of the depsidipeptide derivatives **9a-r** is shown in Scheme 1 below. 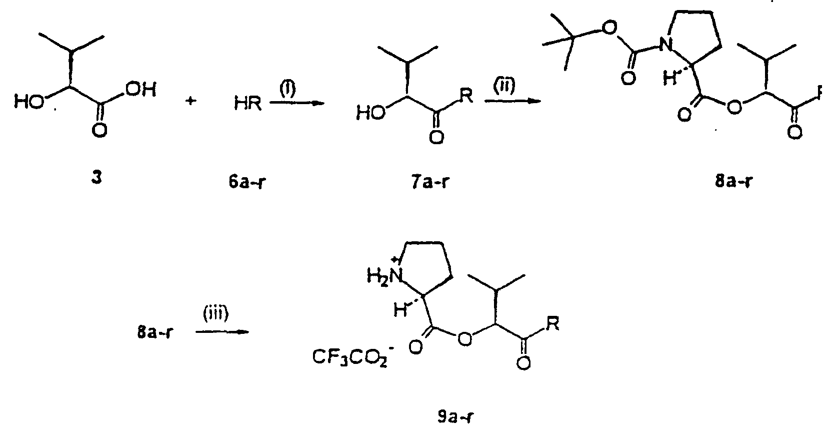 a) R = NHPh; Method B j) R = NH(CH₂)₂-3,4-(CH₃O)₂Ph; Method A b) R = NHCH₂Ph; Method A k) R = NH(CH₂)₂-2-pyridine; Method A c) R = NH(CH₂)₂Ph; Method A l) R = NH(CH₂)₃Ph; Method A d) R = NH(CH₂)₂-4-F-Ph; Method A m) R = L-Phe-OCH₃; Method A e) R = NH(CH₂)₂-4-Cl-Ph; Method A n) R = L-Met-OCH₃; Method A f) R = NH(CH₂)₂-3-Cl-Ph; Method A o) R = L-Pro-OCH₃; Method A g) R = NH(CH₂)₂-2-Cl-Ph; Method A p) R = NH-2-thiazolyl; Method B h) R = NH(CH₂)₂-4-Br-Ph; Method A q) R = NH-2-benzochiazolyl; Method B i) R = NH(CH₂)₂-4-NO₂-Ph;

Method A r) R = NH-3-quinolyl; Method B
(i) Method A: DEPC, NMM, CH₂Cl₂, 0 °C, 2 hours.
   Method B: DCC, HOBt (2 eq.), NMM, THF, 0 °C, 2 hours.
(ii) Boc-L-Pro (1.2 eq.), DCC (1.2 eq.), 4-pyrrolidinopyridine (1.2 eq.), CH₂Cl₂, 23 °C, 18 hours.
   *or* *or* Boc-L-Pro (1.2 eq.), EDC-HCl (1.2 eq.), 4-pyrrolidinopyridine (2.4 eq.), CH₂Cl₂, 23 °C, 18 hours.
(iii) TFA, CH₂Cl₂, 0 °C, 1 hour.

The synthesis of Z- or Boc-pentapeptide derivatives **11a-r** is shown in Scheme 2, below. 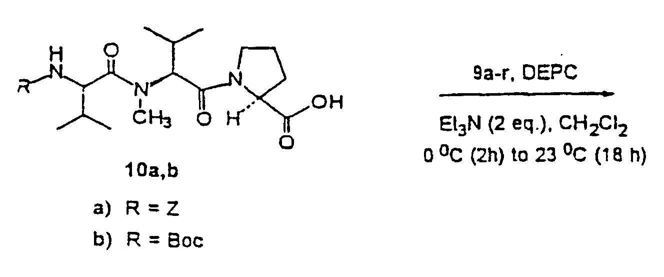 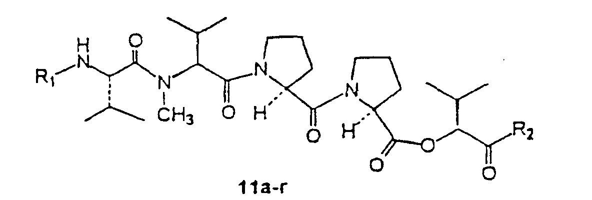 a) R₁ = Z, R₂ = NHPh j) R₁ = Z, R₂ = NH(CH₂)₂-3,4-(CH₃O)₂Ph b) R₁ = Z, R₂ = NHCH₂Ph k) R₁ = Z, R₂ = NH(CH₂)₂-2-pyridine c) R₁ = Z, R₂ = NH(CH₂)₂Ph l) R₁ = Z, R₂ = NH(CH₂)₃Ph d) R₁ = Boc, R₂ = NH(CH₂)₂-4-F-Ph m) R₁ = Z, R₂ = L-Phe-OCH₃ e) R₁ = Z, R₂ = NH(CH₂)₂-4-Cl-Ph n) R₁ = Boc, R₂ = L-Met-OCH₃ f) R₁ = Boc, R₂ = NH(CH₂)₂-3-Cl-Ph o) R₁ = Z, R₂ = L-Pro-OCH₃ g) R₁ = Boc, R₂ = NH(CH₂)₂-2-Cl-Ph p) R₁ = Boc, R₂ = NH-2-thiazolyl h) R₁ = Boc, R₂ = NH(CH₂)₂-4-Br-Ph q) R₁ = Boc, R₂ = NH-2-benzothiazolyl i) R₁ = Boc, R₂ = NH(CH₂)₂-4-NO₂-Ph r) R₁ = Z, R₂ = NH-3-quinolyl

The synthesis of dolastarin 15 derivatives **12a-r** is shown in Scheme 3, below. 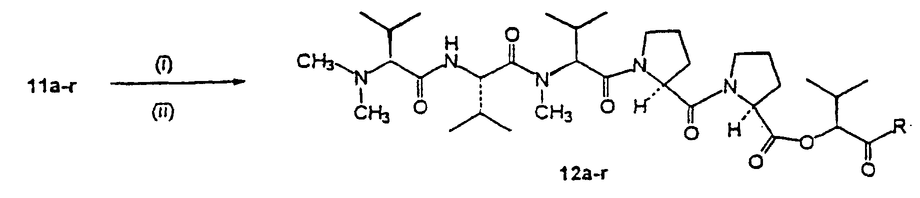 a) R = NHPh; Method A j) R = NH(CH₂)₂-3,4-(CH₃O)₂Ph; Method A b) R = NHCH₂Ph; Method A k) R = NH(CH₂)₂-2-pyridine; Method A c) R = NH(CH₂)₂Ph; Method A l) R = NH(CH₂)₃Ph; Method A d) R = NH(CH₂)₂-4-F-Ph; Method B m) R = L-Phe-OCH₃; Method A e) R = NH(CH₂)₂-4-Cl-Ph; Method C n) R = L-Mer-OCH₃; Method B f) R = NH(CH₂)₂-3-Cl-Ph; Method B o) R = L-Pro-OCH₃; Method A g) R = NH(CH₂)₂-2-Cl-Ph; Method B p) R = NH-2-thiazolyl; Method B h) R = NH(CH₂)₂-4-Br-Ph; Method B q) R = NH-2-benzothiazolyl; Method B i) R = NH(CH₂)₂-4-NO₂-Ph; Method B r) R = NH-3-quinalyl; Method A
(i) Methods A, B, or C.
   Method A: H₂ 10% Pd/C, EtOAc, 18 hours.
   Method B: TFA, CH₂Cl₂, 0°C, 1 hour.
   Method C: 33% HBr/AcOH, 23°C, 30 minutes.
(ii) dolavaline (**4**, 1.2 eq.), DEPC (1.2 eq.), Et₃N (1.2 eq. with Method A; 2.4 eq. with Methods B or C), DEPC (1.2 eq.), CH₂Cl₂, 0°C, 2 hours.

## Claims

1. A dolastatin 15 derivative of formula **12a-r** 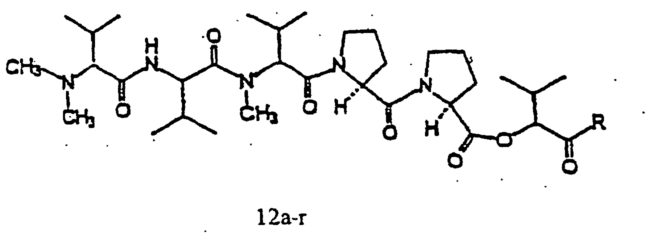 wherein R is selected from the group consisting of:
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-pyridine, NH(CH₂)₃ -Ph, L-Phe-OCH₃, L-Met-OCH₃, L-Pro-OCH₃, NH-2-thiazolyl, NH-2-benzothiazolyl, and NH-3-quinolyl.

2. A method of synthesizing a dolastatin 15 derivative of formula **12a-r** 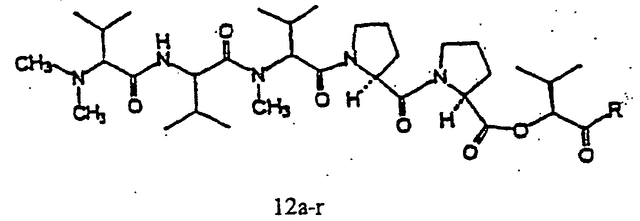 wherein R is selected from the group consisting of:
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-pyridine, NH(CH₂)₃ -Ph, L-Phe-OCH₃, L-Met-OCH₃, L-Pro-OCH₃, NH-2-thiazolyl, NH-2-benzothiazolyl, and NH-3-quinolyl;
said method comprising:
(a) reacting 2-hydroxyisovaleric acid with an amine of formula R-H for a time and under conditions effective to form an amide of formula **7a-r** 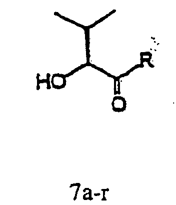 wherein R is as defined above;
(b) reacting the amide of formula **7a-r** with tBoc-L-proline for a time and under conditions effective to form an ester of formula **8a-r** 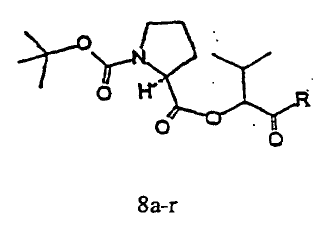
(c) treating the ester of formula **8a-r** with trifluoroacetic acid for a time and under conditions effective to remove the tBoc group from the ester of formula **8a-r**, thereby generating a trifluoroacetic acid salt of formula **9a-r** 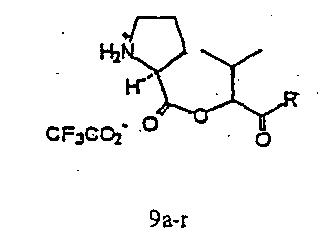
(d) reacting the trifluoroacetic acid salt of formula **9a-r** with the tripeptide of formula **10ab** for a time and under conditions effective to generate the tetrapeptide of formula **11a-r** 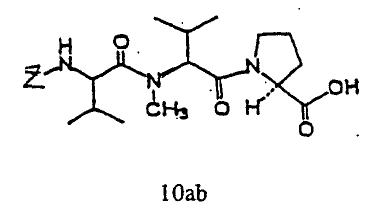 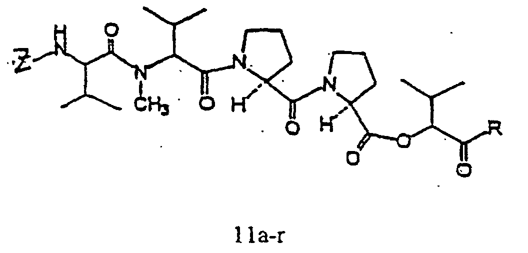 wherein Z is carboxybenzyloxy;
(e) removing the carboxybenzyloxy group from the tetrapeptide of formula **11a-r** to form a deprotected tetrapeptide, and reacting said deprotected tetrapeptide with N, N'-dimethylvaline for a time and under conditions effective to generate said dolastatin 15 derivative of formula **12a-r**; and
(f) recovering said dolastatin 15 derivative of formula **12a-r**.

3. A method of synthesizing a dolastatin 15 derivative according to claim 2 wherein step (a) is conducted in the presence of 4-methylmorpholine (NMM) and diethylphosphorocyanidate (DEPC).

4. A method according to claim 2 wherein step (a) is conducted in the presence of 1-hydroxybenzotriazole (HOBt), 4-methylmorpholine (NMM) and N, N'-dicyclohexylcarbodiimide (DCC).

5. A method according to claim 2 wherein step (b) is conducted in the presence of N,N'-dicyclohexylcarbodiimide (DCC) and 4-pyrrohdinopyridine.

6. A method according to claim 2 wherein step (b) is conducted in the presence of 1-ethyl-3(3-dimethylaminopropyl) carbodiimide hydrochloride (EDC-HCl) and 4-pyrrolidinopyridine.

7. A method according to claim 2 wherein step (d) is conducted in the presence of Et₃N and diethylphosphorocyanidate (DEPC).

8. A method according to claim 2 wherein in step (e) the tetrapeptide of formula **11a-r** is deprotected in a solution of a tetrapeptide of formula **11a-r** in EtOAc to which 10% Pd/C catalyst is added.

9. A method according to claim 2 wherein in step (e) the tetrapeptide of formula **11a-r** is deprotected in a solution of a tetrapeptide of formula **11a-r** in CH₂Cl₂ to which 10% Pd/C catalyst is added.

10. A method according to claim 2 wherein in step (e) the tetrapeptide of formula **11a-r** is deprotected in the presence of a solution of a tetrapeptide of formula **11a-r** suspended in glacial acetic acid to which 33% HBr/AcOH is added.

11. A method according to claim 2 wherein in step (e) the reaction of the tetrapeptide of formula **11a-r** with N, N'-dimethylvaline is conducted in the presence of Et₃N and diethylphosphorocyanidate (DEPC).

12. A method of synthesizing a dolastatin 15 derivative of formula **12a-r** 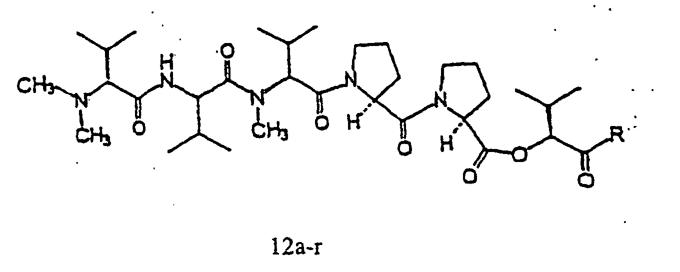 wherein R is selected from the group consisting of :
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-pyridine, NH(CH₂)₃ -Ph, L-Phe-OCH₃, L-Met-OCH₃ , L-Pro-OCH₃, NH-2-thiazolyl, NH-2-benzothiazolyl, and NH-3-quinolyl;
said method comprising:
(a) reacting 2-hydroxyisovaleric acid with an amine of formula R-H for a time and under conditions effective to form an amide of formula **7a-r** 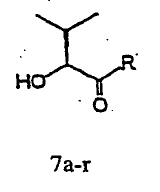 wherein R is as defined above;
(b) reacting the amide of formula **7a-r** with tBoc-L-proline for a time and under conditions effective to form an ester of formula **8a-r** 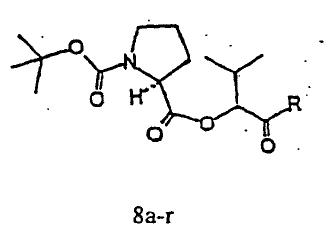
(c) treating the ester of formula **8a-r** with trifluoracetic acid for a time and under conditions effective to remove the tBoc group from the ester of formula **8a-r,** thereby generating a trifluoroacetic acid salt of formula **9a-r** 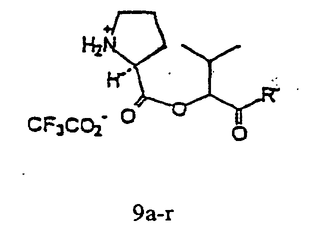
(d) reacting the trifluoroacetic acid salt of formula **9a-r** with the tripeptide of formula **10ab** for a time and under conditions effective to generate the tetrapeptide of formula **11a-r** 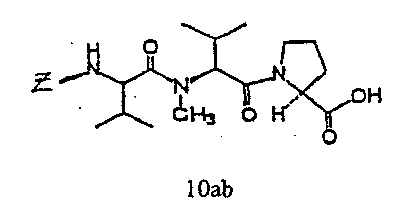 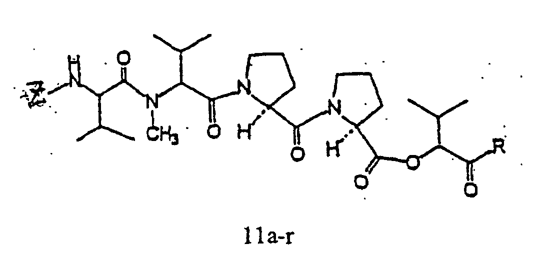 wherein Z is tBoc;
(e) removing the tBoc group from the tetrapeptide of formula **11a-r** to form a deprotected tetrapeptide, and reacting said deprotected tetrapeptide with N, N'-dimethylvaline for a time and under conditions effective to generate said dolastatin 15 derivative of formula **12a-r;** and
(f) recovering said dolastatin 15 derivative of formula **12a-r**.

13. A method of synthesizing a dolastatin 15 derivative according to claim 12 wherein step (a) is conducted in the presence of 4-methylmorpholine (NMM) and diethylphosphorocyanidate (DEPC).

14. A method according to claim 12 wherein step (a) is conducted in the presence of 1-hydroxybenzotriazole (HOBt), 4-methylmorpholine (NMM) and N, N'-dicyclohexylcarbodiimide (DCC).

15. A method according to claim 12 wherein step (b) is conducted in the presence of N,N'-dicyclohexylcarbodiimide (DCC) and 4-pyrrolidinopyridine.

16. A method according to claim 12 wherein step (b) is conducted in the presence of 1-ethyl-3(3-dimethylaminopropyl) carbodiimide hydrochloride (EDC-HCl) and 4-pyrrolidinopyridine.

17. A method according to claim 12 wherein step (d) is conducted in the presence of Et₃N and diethylphosphorocyanidate (DEPC).

18. A method according to claim 12 wherein in step (e) the tetrapeptide of formula **11a-r** is deprotected in the presence of a solution of a tetrapeptide of formula **11a-r** in EtOAc to which 10% Pd/C catalyst is added.

19. A method according to claim 12 wherein in step (e) the tetrapeptide of formula **11a-r** is deprotected in the presence of a solution of a tetrapeptide of formula **11a-r** in CH₂Cl₂ to which 10% Pd/C catalyst is added.

20. A method according to claim 12 wherein in step (e) the tetrapeptide of formula **11a-r** is deprotected in the presence of a solution of a tetrapeptide of formula **11a-r** suspended in glacial acetic acid to which 33% HBr/AcOH is added.

21. A method according to claim 12 wherein in step (e) the reaction of the tetrapeptide of formula **11a-r** with N, N' - dimethylvaline is conducted in the presence of Et₃N and diethylphosphorocyanidate (DEPC).

22. The dolastatin 15 derivative of claim 1, wherein R=NH(CH₂)₂Ph.

23. Compounds of claims 1 or 22 for use in medicine.

24. Compounds according to claim 23 wherein the compounds are used to treat cancer and/or inhibit tubulin polymerisation and/or inhibit growth of bacteria.

25. A use of compounds according to claims 1 or 22 in the manufacture of a medicament for the treatment of cancer and/or the inhibition of tubulin polymerisation and/or the inhibition of the growth of bacteria.

26. A use according to claims 24 or 25 wherein the cancer is leukaemia.

27. A use according to claims 24 or 25 wherein the cancer is Burkitt Lymphoma.

28. A use according to claims 24 or 25 wherein the bacteria is *Erwinia carotovora*.

## Patentansprüche

1. Dolastatin-15-Derivat der Formel 12a-r 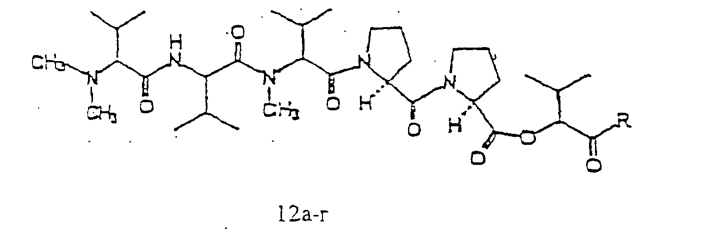 worin R ausgewählt ist aus der Gruppe von:
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-Pyridin, NH(CH₂)₃-Ph, L-Phe-OCH₃, L-Met-OCH₃, L-Pro-OCH₃, NH-2-Thiazolyl, NH-2-Benzothiazolyl und NH-3-Chinolyl.

2. Verfahren zur Synthese eines Dolastatin-15-Derivats der Formel 12a-r 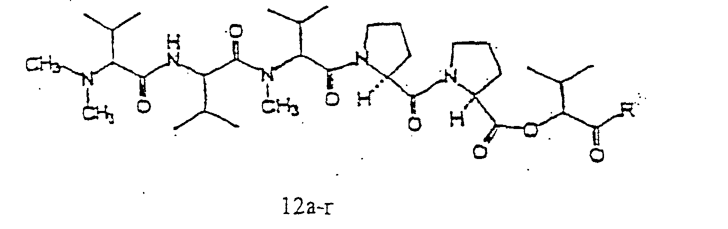 worin R ausgewählt ist aus der Gruppe von:
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-Pyridin, NH(CH₂)₃-Ph, L-Phe-OCH₃, L-Met-OCH₃, L-Pro-OCH₃, NH-2-Thiazolyl, NH-2-Benzothiazolyl und NH-3-Chinolyl;
wobei das Verfahren umfasst:
(a) Umsetzen von 2-Hydroxyisovaleriansäure mit einem Amin der Formel R-H über einen Zeitraum und unter Bedingungen, die zur Bildung eines Amids der Formel 7a-r 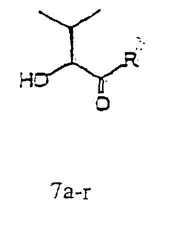 worin R wie im vorhergehenden definiert ist, wirksam sind;
(b) Umsetzen des Amids der Formel 7a-r mit tBoc-L-Prolin über einen Zeitraum und unter Bedingungen, die zur Bildung eines Esters der Formel 8a-r 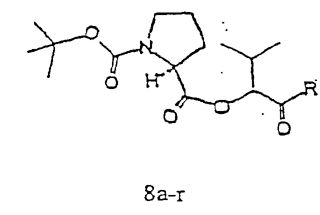 wirksam sind;
(c) Behandeln des Esters der Formel 8a-r mit Trifluoressigsäure über einen Zeitraum und unter Bedingungen, die zur Entfernung der tBoc-Gruppe von dem Ester der Formel 8a-r wirksam sind, wodurch ein Trifluoressigsäuresalz der Formel 9a-r 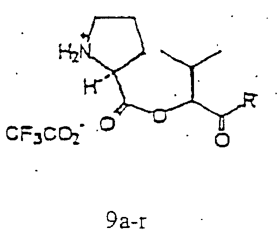 gebildet wird;
(d) Umsetzen des Trifluoressigsäuresalzes der Formel 9a-r mit dem Tripeptid der Formel 10ab über einen Zeitraum und unter Bedingungen, die zur Erzeugung des Tetrapeptids der Formel 11a-r wirksam sind; 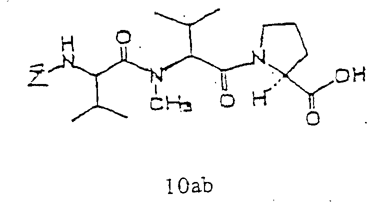 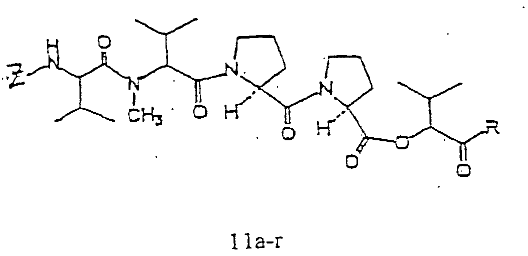 worin Z Carboxybenzyloxy bedeutet;
(e) Entfernen der Carboxybenzyloxygruppe von dem Tetrapeptid der Formel 11a-r unter Bildung eines entschützten Tetrapeptids und Umsetzen des entschützten Tetrapeptids mit N,N'-Dimethylvalin über einen Zeitraum und unter Bedingungen, die zur Erzeugung des Dolastatin-15-Derivats der Formel 12a-r wirksam sind; und
(f) Gewinnen des Dolastatin-15-Derivats der Formel 12a-r.

3. Verfahren zur Synthese eines Dolastatin-15-Derivats nach Anspruch 2, wobei die Stufe (a) in Gegenwart von 4-Methylmorpholin (NMM) und Diethylphosphorocyanidat (DEPC) durchgeführt wird.

4. Verfahren nach Anspruch 2, wobei die Stufe (a) in Gegenwart von 1-Hydroxybenzotriazol (HOBt), 4-Methylmorpholin (NMM) und N,N'-Dicyclohexylcarbodiimid (DCC) durchgeführt wird.

5. Verfahren nach Anspruch 2, wobei die Stufe (b) in Gegenwart von N,N'-Dicyclohexylcarbodiimid (DCC) und 4-Pyrrolidinopyridin durchgeführt wird.

6. Verfahren nach Anspruch 2, wobei die Stufe (b) in Gegenwart von 1-Ethyl-3-(3-dimethylaminopropyl)carbodiimidhydrochlorid (EDC-HCl) und 4-Pyrrolidinopyridin durchgeführt wird.

7. Verfahren nach Anspruch 2, wobei die Stufe (b) in Gegenwart von Et₃N und Diethylphosphorocyanidat (DEPC) durchgeführt wird.

8. Verfahren nach Anspruch 2, wobei in der Stufe (e) das Tetrapeptid der Formel 11a-r in einer Lösung eines Tetrapeptids der Formel 11a-r in EtOAc, der ein 10 % Pd/C-Katalysator zugesetzt wurde, entschützt wird.

9. Verfahren nach Anspruch 2, wobei in der Stufe (e) das Tetrapeptid der Formel 11a-r in einer Lösung eines Tetrapeptids der Formel 11a-r in CH₂Cl₂, der ein 10 % Pd/C-Katalysator zugesetzt wurde, entschützt wird.

10. Verfahren nach Anspruch 2, wobei in der Stufe (e) das Tetrapeptid der Formel 11a-r in Gegenwart einer Lösung eines Tetrapeptids der Formel 11a-r, das in Eisessig suspendiert ist, unter Zusatz von 33 % HBr/AcOH entschützt wird.

11. Verfahren nach Anspruch 2, wobei in der Stufe (e) die Umsetzung des Tetrapeptids der Formel 11a-R mit N,N'-Dimethylvalin in Gegenwart von Et₃N und Diethylphosphorocyanidat (DEPC) durchgeführt wird.

12. Verfahren zur Synthese eines Dolastatin-15-Derivats der Formel 12a-r 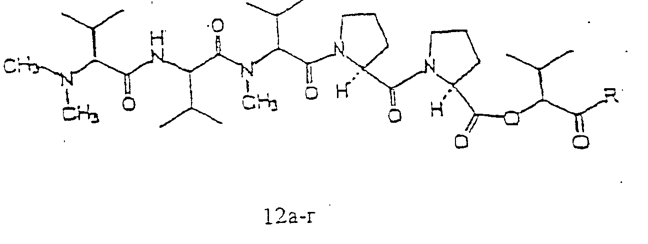 worin R ausgewählt ist aus der Gruppe von:
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-Pyridin, NH(CH₂)₃-Ph, L-Phe-OCH₃, L-Met-OCH₃, L-Pro-OCH₃, NH-2-Thiazolyl, NH-2-Benzothiazolyl und NH-3-Chinolyl;
wobei das Verfahren umfasst:
(a) Umsetzen von 2-Hydroxyisovaleriansäure mit einem Amin der Formel R-H über einen Zeitraum und unter Bedingungen, die zur Bildung eines Amids der Formel 7a-r 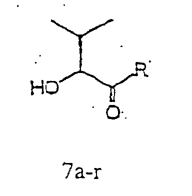 worin R wie im vorhergehenden definiert ist, wirksam sind;
(b) Umsetzen des Amids der Formel 7a-r mit tBoc-L-Prolin über einen Zeitraum und unter Bedingungen, die zur Bildung eines Esters der Formel 8a-r 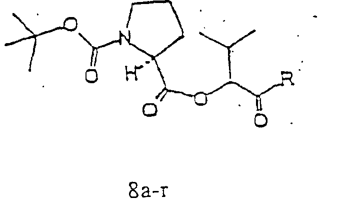 wirksam sind;
(c) Behandeln des Esters der Formel 8a-r mit Trifluoressigsäure über einen Zeitraum und unter Bedingungen, die zur Entfernung der tBoc-Gruppe von dem Ester der Formel 8a-r wirksam sind, wodurch ein Trifluoressigsäuresalz der Formel 9a-r 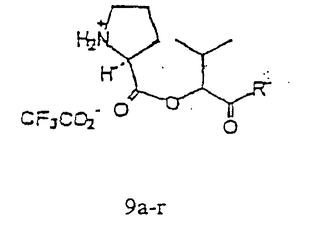 gebildet wird;
(d) Umsetzen des Trifluoressigsäuresalzes der Formel 9a-r mit dem Tripeptid der Formel 10ab über einen Zeitraum und unter Bedingungen, die zur Erzeugung des Tetrapeptids der Formel 11a-r wirksam sind; 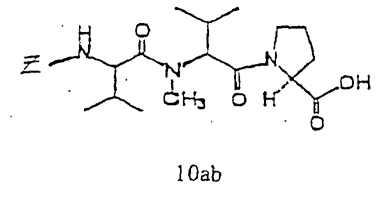 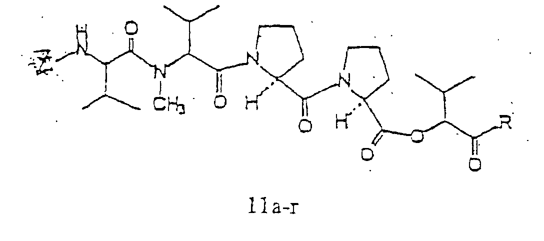 worin Z tBoc bedeutet;
(e) Entfernen der tBoc-Gruppe von dem Tetrapeptid der Formel 11a-r unter Bildung eines entschützten Tetrapeptids und Umsetzen des entschützten Tetrapeptids mit N,N'-Dimethylvalin über einen Zeitraum und unter Bedingungen, die zur Erzeugung des Dolastatin-15-Derivats der Formel 12a-r wirksam sind; und
(f) Gewinnen des Dolastatin-15-Derivats der Formel 12a-r.

13. Verfahren zur Synthese eines Dolastatin-15-Derivats nach Anspruch 12, wobei die Stufe (a) in Gegenwart von 4-Methylmorpholin (NMM) und Diethylphosphorocyanidat (DEPC) durchgeführt wird.

14. Verfahren nach Anspruch 12, wobei die Stufe (a) in Gegenwart von 1-Hydroxybenzotriazol (HOBt), 4-Methylmorpholin (NMM) und N,N'-Dicyclohexylcarbodiimid (DCC) durchgeführt wird.

15. Verfahren nach Anspruch 12, wobei die Stufe (b) in Gegenwart von N,N'-Dicyclohexylcarbodiimid (DCC) und 4-Pyrrolidinopyridin durchgeführt wird.

16. Verfahren nach Anspruch 12, wobei die Stufe (b) in Gegenwart von 1-Ethyl-3-(3-dimethylaminopropyl)carbodiimidhydrochlorid (EDC-HCl) und 4-Pyrrolidinopyridin durchgeführt wird.

17. Verfahren nach Anspruch 12, wobei die Stufe (d) in Gegenwart von Et₃N und Diethylphosphorocyanidat (DEPC) durchgeführt wird.

18. Verfahren nach Anspruch 12, wobei in der Stufe (e) das Tetrapeptid der Formel 11a-r in Gegenwart einer Lösung eines Tetrapeptids der Formel 11a-r in EtOAc, der ein 10 % Pd/C-Katalysator zugesetzt wurde, entschützt wird.

19. Verfahren nach Anspruch 12, wobei in der Stufe (e) das Tetrapeptid der Formel 11a-r in Gegenwart einer Lösung eines Tetrapeptids der Formel 11a-r in CH₂Cl₂, der ein 10 % Pd/C-Katalysator zugesetzt wurde, entschützt wird.

20. Verfahren nach Anspruch 12, wobei in der Stufe (e) das Tetrapeptid der Formel 11a-r in Gegenwart einer Lösung eines Tetrapeptids der Formel 11a-r, das in Eisessig suspendiert ist, unter Zusatz von 33 % HBr/AcOH entschützt wird.

21. Verfahren nach Anspruch 12, wobei in der Stufe (e) die Umsetzung des Tetrapeptids der Formel 11a-r mit N,N'-Dimethylvalin in Gegenwart von Et₃N und Diethylphosphorocyanidat (DEPC) durchgeführt wird.

22. Dolastatin-15-Derivat nach Anspruch 1, wobei R = NH(CH₂)₂Ph.

23. Verbindungen nach Anspruch 1 oder 22, zur Verwendung in der Medizin.

24. Verbindungen nach Anspruch 23, wobei die Verbindungen zur Behandlung von Krebs und/oder zur Hemmung der Tubulinpolymerisation und/oder zur Hemmung des Wachstums von Bakterien verwendet werden.

25. Verwendung von Verbindungen nach den Ansprüchen 1 oder 22 bei der Herstellung eines Medikaments zur Behandlung von Krebs und/oder zur Hemmung der Tubulinpolymerisation und/oder zur Hemmung des Wachstums von Bakterien.

26. Verwendung nach den Ansprüchen 24 oder 25, wobei der Krebs Leukämie ist.

27. Verwendung nach den Ansprüchen 24 oder 25, wobei der Krebs Burkitt-Lymphom ist.

28. Verwendung nach den Ansprüchen 24 oder 25, wobei das Bakterium *Erwinia carotovora* ist.

## Revendications

1. Dérivé 15 de dolastatine de formule 12a-r : 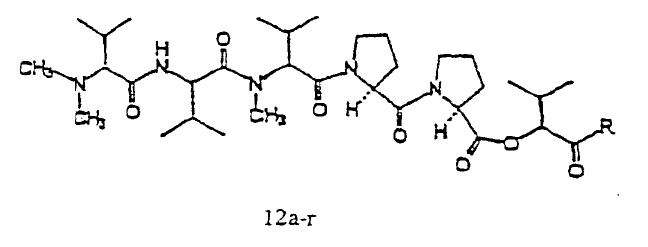 dans laquelle R est choisi dans le groupe constitué de :
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-pyridine, NH(CH₂)₃-Ph, L-Phé-OCH₃, L-Mét-OCH₃, L-Pro-OCH₃, NH-2-thiazolyle, NH-2-benzothiazolyle et NH-3-quinoléyle.

2. Procédé de synthèse d'un dérivé 15 de dolastatine de formule 12a-r 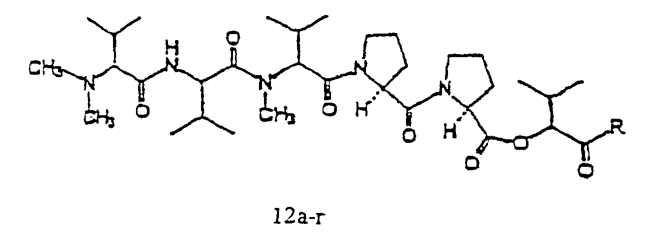 dans laquelle R est choisi dans le groupe constitué de :
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-pyridine, NH(CH₂)₃-Ph, L-Phé-OCH₃, L-Mét-OCH₃, L-Pro-OCH₃, NH-2-thiazolyle, NH-2-benzothiazolyle et NH-3-quinoléyle ;
ledit procédé comprenant les étapes consistant à :
(a) faire réagir l'acide 2-hydroxyisovalérique avec une amine de formule R-H pendant une durée et dans des conditions efficaces pour former un amide de formule 7a-r 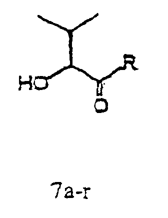 dans laquelle R est tel que défini ci-dessus ;
(b) faire réagir l'amide de formule 7a-r avec t-Boc-L-proline pendant une durée et dans des conditions efficaces pour former un ester de formule 8a-r 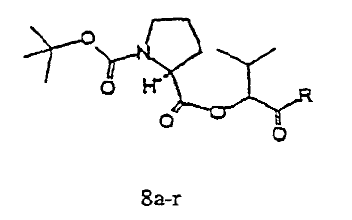
(c) traiter l'ester de formule 8a-r avec de l'acide trifluoroacétique pendant une durée et dans des conditions efficaces pour éliminer le groupe tBoc de l'ester de formule 8a-r, produisant ainsi un sel d'acide trifluoroacétique de formule 9a-r 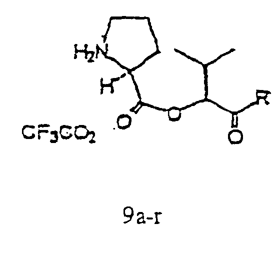
(d) faire réagir le sel d'acide trifluoroacétique de formule 9a-r avec le tripeptide de formule 10ab pendant une durée et dans des conditions efficaces pour produire le tétrapeptide de formule 11a-r 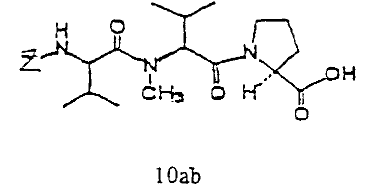 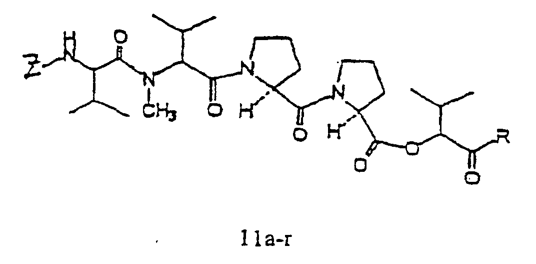 dans lesquelles Z représente un groupe carboxybenzyloxy ;
(e) éliminer le groupe carboxybenzyloxy du tétrapeptide de formule 11 a-r pour former un tétrapeptide déprotégé, et faire réagir ledit tétrapeptide déprotégé avec de la N,N'-diméthylvaline pendant une durée et dans des conditions efficaces pour produire ledit dérivé 15 de dolastatine de formule 12a-r ; et
(f) récupérer ledit dérivé 15 de dolastatine de formule 12a-r.

3. Procédé de synthèse d'un dérivé 15 de dolastatine selon la revendication 2, dans lequel l'étape (a) est réalisée en présence de 4-méthylmorpholine (NMM) et de diéthylphosphorocyanidate (DEPC).

4. Procédé selon la revendication 2, dans lequel l'étape (a) est réalisée en présence de 1-hydroxybenzotriazole (HOBt), de 4-méthylmorpholine (NMM) et de N-N'-dicyclohexylcarbodiimide (DCC).

5. Procédé selon la revendication 2, dans lequel l'étape (b) est réalisée en présence de N,N'-dicyclohexylcarbodiimide (DCC) et de 4-pyrrolidinopyridine.

6. Procédé selon la revendication 2, dans lequel l'étape (b) est réalisé en présence de chlorhydrate de 1-éthyl-3(3-diméthylaminopropyle)-carbodiimide (EDC-HCl) et de 4-pyrrolidinopyridine.

7. Procédé selon la revendication 2, dans lequel l'étape (d) est réalisée en présence de Et₃N et de diéthylphosphorocyanidate (DEPC).

8. Procédé selon la revendication 2, dans lequel, à l'étape (e), le tétrapeptide de formule 11a-r est déprotégé dans une solution d'un tétrapeptide de formule 11a-r dans EtOAc à laquelle on ajoute un catalyseur Pd à 10%/C.

9. Procédé selon la revendication 2, dans lequel, à l'étape (e), le tétrapeptide de formule 11a-r est déprotégé dans une solution d'un tétrapeptide de formule 11a-r dans CH₂Cl₂ à laquelle on ajoute un catalyseur Pd à 10%/C.

10. Procédé selon la revendication 2, dans lequel, à l'étape (e), le tétrapeptide de formule 11a-r est déprotégé en présence d'une solution d'un tétrapeptide de formule 11a-r mis en suspension dans de l'acide acétique glacial à laquelle on ajoute HBr à 33%/AcOH.

11. Procédé selon la revendication 2, dans lequel, à l'étape (e), la réaction du tétrapeptide de formule 11a-r avec de la N,N'-diméthylvaline est réalisée en présence de Et₃N et de diéthylphosphorocyanidate (DEPC).

12. Procédé de synthèse d'un dérivé de dolastatine de formule 12a-r 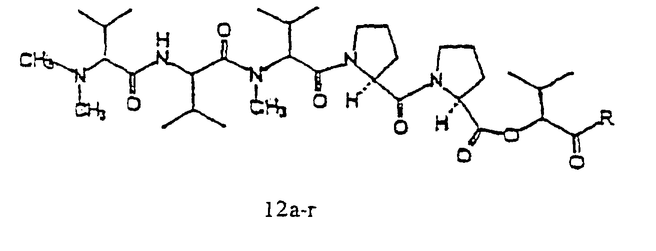 dans laquelle R est choisi dans le groupe constitué de :
NHPh, NHCH₂Ph, NH(CH₂)₂Ph, NH(CH₂)₂-4-F-Ph, NH(CH₂)₂-4-Cl-Ph, NH(CH₂)₂-3-Cl-Ph, NH(CH₂)₂-2-Cl-Ph, NH(CH₂)₂-4-Br-Ph, NH(CH₂)₂-4-NO₂-Ph, NH(CH₂)₂-3,4-(CH₃O)₂Ph, NH(CH₂)₂-2-pyridine, NH(CH₂)₃-Ph, L-Phé-OCH₃, L-Mét-OCH₃, L-Pro-OCH₃, NH-2-thiazolyle, NH-2-benzothiazolyle et NH-3-quinoléyle ;
ledit procédé comprenant les étapes consistant à :
(a) faire réagir l'acide 2-hydroxyisovalérique avec une amine de formule R-H pendant une durée et dans des conditions efficaces pour former un amide de formule 7a-r 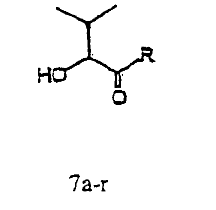 dans laquelle R est tel que défini ci-dessus ;
(b) faire réagir l'amide de formule 7a-r avec t-Boc-L-proline pendant une durée et dans des conditions efficaces pour former un ester de formule 8a-r 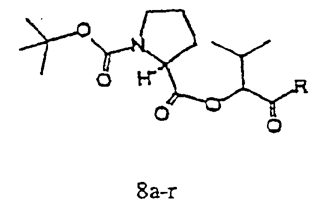
(c) traiter l'ester de formule 8a-r avec de l'acide trifluoroacétique pendant une durée et dans des conditions efficaces pour éliminer le groupe tBoc de l'ester de formule 8a-r, produisant ainsi un sel d'acide trifluoroacétique de formule 9a-r 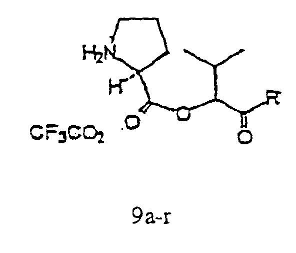
(d) faire réagir le sel d'acide trifluoroacétique de formule 9a-r avec le tripeptide de formule 10ab pendant une durée et dans des conditions efficaces pour produire le tétrapeptide de formule 11a-r 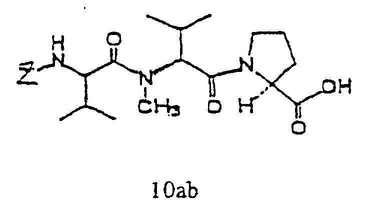 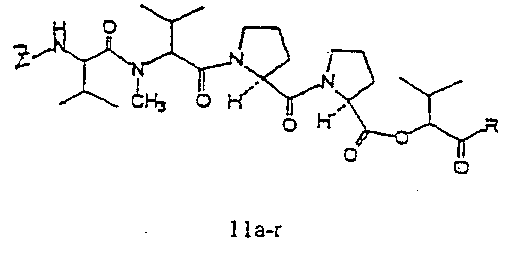 dans lesquelles Z représente tBoc ;
(e) éliminer le groupe tBoc du tétrapeptide de formule 11 a-r pour former un tétrapeptide déprotégé, et faire réagir ledit tétrapeptide déprotégé avec la N,N'-diméthylvaline pendant une durée et dans des conditions efficaces pour produire ledit dérivé 15 de dolastatine de formule 12a-r ; et
(f) récupérer ledit dérivé 15 de dolastatine de formule 12a-r.

13. Procédé de synthèse d'un dérivé 15 de dolastatine selon la revendication 12, dans lequel l'étape (a) est réalisée en présence de 4-méthylmorpholine (NMM) et de diéthylphosphorocyanidate (DEPC).

14. Procédé selon la revendication 12, dans lequel l'étape (a) est réalisée en présence de 1-hydroxybenzotriazole (HOBt), de 4-méthylmorpholine (NMM) et de N-N'-dicyclohexylcarbodiimide (DCC).

15. Procédé selon la revendication 12, dans lequel l'étape (b) est réalisée en présence de N,N'-dicyclohexylcarbodiimide (DCC) et de 4-pyrrolidinopyridine.

16. Procédé selon la revendication 12, dans lequel l'étape (b) est réalisée en présence de chlorhydrate de 1-éthyl-3(3-diméthylaminopropyle)-carbodiimide (EDC-HCl) et de 4-pyrrolidinopyridine.

17. Procédé selon la revendication 12, dans lequel l'étape (d) est réalisée en présence de Et₃N et de diéthylphosphorocyanidate (DEPC).

18. Procédé selon la revendication 12, dans lequel, à l'étape (e), le tétrapeptide de formule 11a-r est déprotégé dans une solution d'un tétrapeptide de formule 11a-r dans EtOAc à laquelle on ajoute un catalyseur Pd à 10%/C.

19. Procédé selon la revendication 12, dans lequel, à l'étape (e), le tétrapeptide de formule 11a-r est déprotégé dans une solution d'un tétrapeptide de formule 11a-r dans CH₂Cl₂ à laquelle on ajoute un catalyseur Pd à 10%/C.

20. Procédé selon la revendication 12, dans lequel, à l'étape (e), le tétrapeptide de formule 11a-r est déprotégé dans une solution d'un tétrapeptide de formule 11a-r mis en suspension dans de l'acide acétique glacé à laquelle on ajoute HBr à 33%/AcOH.

21. Procédé selon la revendication 12, dans lequel, à l'étape (e), la réaction du tétrapeptide de formule 11a-r avec de la N,N'-diméthylvaline est réalisée en présence de Et₃N et de diéthylphosphorocyanidate (DEPC).

22. Dérivé 15 de dolastatine selon la revendication 1, dans lequel R = NH(CH₂)₂Ph.

23. Composés selon la revendication 1 ou 22 pour une utilisation médicale.

24. Composés selon la revendication 23 dans lesquels les composés sont utilisés pour traiter un cancer et/ou inhiber la polymérisation de tubuline et/ou inhiber la croissance de bactéries.

25. Utilisation de composés selon la revendication 1 ou 22 dans la préparation d'un médicament pour le traitement d'un cancer et/ou l'inhibition de la polymérisation de tubuline et/ou l'inhibition de la croissance de bactéries.

26. Utilisation selon la revendication 24 ou 25 dans laquelle le cancer est une leucémie.

27. Utilisation selon la revendication 24 ou 25 dans laquelle le cancer est le lymphome de Burkitt.

28. Utilisation selon la revendication 24 ou 25, dans laquelle les bactéries sont *Erwinia carotovora*.
